# EUROPEAN PATENT SPECIFICATION

(11) **EP 1 915 165 B1**
(45) Date of publication and mention of the grant of the patent: **02.07.2014**
(21) Application number: 06789412.1
(22) Date of filing: 04.08.2006
(51) Int. Cl.: A61F 13/00, C12N 5/00, C12N 5/02, C12N 5/095

(54) **TOPOGRAPHICAL TEMPLATING OF POLYMERIC MATERIALS USING CELLULAR MORPHOLOGY**
TOPOGRAPHISCHES SCHABLONIEREN POLYMERER MATERIALIEN MIT ZELL-MORPHOLOGIE
MODELISATION TOPOGRAPHIQUE DE MATIERES POLYMERES AU MOYEN D'UNE MORPHOLOGIE CELLULAIRE

(30) Priority: 12.08.2005 US 707912 P
(43) Date of publication of application: 30.04.2008
(73) Proprietor: Brown University, Providence, RI 02912 (US)
(72) Inventor: HOFFMAN-KIM, Diane, Providence, RI 02906 (US); BRUDER, Jan, M., Providence, RI 02912 (US)
(74) Representative: Kirkham, Nicholas Andrew
(86) International application number: PCT/US2006/030466
(87) International publication number: WO 2007/021590

(56) References cited:
- WO-A2-02/101028
- US-A- 5 681 568
- US-A- 5 885 829
- US-A1- 2004 096 476
- US-B1- 6 855 329
- FLEMMING R G ET AL: "EFFECTS OF SYNTHETIC MICRO-AND NANO-STRUCTURED SURFACES ON CELL BEHAVIOR" BIOMATERIALS, ELSEVIER SCIENCE PUBLISHERS BV., BARKING, GB, vol. 20, 1 March 1999 (1999-03-01), pages 573-588, XP002926697 ISSN: 0142-9612
- MOYA S E ET AL: "COMPOSITE LIPID POLYELECTROLYTE CAPSULES TEMPLATED ON RED BLOOD CELLS: FABRICATION AND STRUCTURAL CHARACTERISATION" MEDICAL AND BIOLOGICAL ENGINEERING AND COMPUTING, SPRINGER, HEILDELBERG, DE, vol. 41, no. 4, 1 July 2003 (2003-07-01), pages 504-508, XP001047280 ISSN: 0140-0118
- XIA Y.: 'Soft Lithography' ANGEW. CHEM. INT. ED. vol. 37, 1998, pages 550 - 575, XP002149045
- MATSUZAWA M. ET AL.: 'Effects of Organosilane Monolayer Films on the Geometrical Guidance of CNS Neurons' LANGMUIR vol. 14, no. 18, 1998, pages 5133 - 5138
- M'BARECK C O ET AL: "Poly (acrylic acid) and poly (sodium styrenesulfonate) compatibility by Fourier transform infrared and differential scanning calorimetry", POLYMER, ELSEVIER SCIENCE PUBLISHERS B.V, GB, vol. 45, no. 12, 1 May 2004 (2004-05-01), pages 4181-4187, XP004508703, ISSN: 0032-3861, DOI: 10.1016/J.POLYMER.2004.03.044
- DATABASE COMPENDEX [Online] ENGINEERING INFORMATION, INC., NEW YORK, NY, US 21 July 2010 SHAO L ET AL: 'Thermochemical properties of free-standing electrostatic layer-by-layer assemblies containing poly(allylamine hydrochloride) and poly(acrylic acid)' Database accession no. E20102913082509 & SHAO L ET AL: "Thermochemical properties of free-standing electrostatic layer-by-layer assemblies containing poly(allylamine hydrochloride) and poly(acrylic acid)", SOFT MATTER 20100721 ROYAL SOCIETY OF CHEMISTRY GBR, vol. 6, no. 14, 21 July 2010 (2010-07-21), pages 3363-3369, DOI: 10.1039/C0SM00082E

## Description

### FIELD OF THE INVENTION

The invention relates to materials and methods for the topographical templating of polymeric materials and substrates that can be employed to induce and stimulate guided cell growth.

### BACKGROUND OF THE INVENTION

Polymers, natural and synthetic, have been employed in drug delivery and in biomedical engineering for many years. In biomedical engineering, the list of such usable polymers is extensive. So is the list of shapes, sizes and patterns proposed by researchers in cell and tissue engineering. The desire has been to create surfaces and forms that influence cell adhesion, spreading, growth and organization in a manner which induces and stimulates controlled and selective growth and differentiation, or regeneration, of cells and tissues.

For example, in the field of nerve regeneration, it has been proposed to employ substrates having surface containing grooves in the interior surface of guidance conduits, with chemical, cellular or electrical "cues," known as "nerve growth guidance factors", provided in the grooves to obtain the desired nerve growth rates to regain nerve functionality. See for example United States Patent No. 6,676,675 and United States Patent Publication No. 2002/0051806 in the name of Mallapragada et al. The growing tips of an extending axon (the "growth cone") explores the surrounding environment transiently contacting neurons, glia and their associated molecules in order to generate directed axon growth. Various extracellular cues affect the growth cone's behavior. Such cues include cell adhesion molecules, extracellular matrix molecules, neurotrophic factors, chemoattractants, chemorepellants and the eph family of molecules. Preferred materials for these "micropatterned substrates" include poly(D,L-lactide) (PDLA), copolymers of lactic and glycolic acids (PLGA), glycolide trimethylene carbonate, polyester, polyglycolic acid, polyglycolic-acid mesh coated with collagen, collagen, polylactic acid, poly(organo)phosphazine, polyorthoester, copolymers of collagen and glycosaminoglycans, copolymers of L-lactide and ε-caprolactone, mixtures of polyurethane and polylactic acid and polyimides and polystyrene.

Additional work in the field of nerve regeneration has concentrated on controlling the placement and orientation of Schwann cells. It has been found that Schwann cells, which pay a key role in neuronal regeneration, predominantly attach and elongate on laminin stripes in preference to bovine serum albumin stripes and organize into multicellular aggregates oriented with the laminin pattern on the substrate in a time-dependent manner. Thompson and Buettner, Annals of Biomedical Engineering 32 (2004) 1120-30 and Tissue Engineering 7 (2001) 247-65. Poly(L-lactic acid) (PLLA) conduits containing Schwann cells in a collagen matrix have been implanted into 12 mm sciatic nerve defects in rats, but peripheral nerve regeneration was not functionally enhanced by implantation of these conduits. Evans et al., Biomaterials 23 (2002) 841-848.

Mats made from individual fibers of fibronectin, an extracellular matrix cell adhesion glycoprotein, have been used to promote alignment by contact guidance of human dermal fibroblasts, neurites, macrophages and epitenon fibroblasts. Such fibers have also been employed to orient and stimulate the growth of regenerating axons and Schwann cells in the rat model and have been shown to act as a depot for local, sustained-release delivery of neurotrophins that improve peripheral nerve regeneration. Ahmed and Brown, in Cell Motility and the Cytoskeleton 42 (1999) 331-43, suggest that the use of fibronectin fibers and mats as "scaffolds" would have a duel positive effect in neuronal cell guidance as well as in stimulating cell migration.

United States Patent Publication 2001/0031974 in the name of Hadlock and Sundback discloses a spiral neural regeneration conduit composed of a porous biocompatible support having an inner surface and an outer surface onto which neurological cells can be cultured and into with neurotrophic agents can be deposited. Suitable disclosed polymerics include polyhydroxyalkanoates such as polyhydroxybutyric acid, polyesters such as polyglycolic acid, copolymers of glycolic acid and lactic acid; copolymers of lactic acid and ε-aminocaproic acid, polycaprolactones, polydesoxazon, copolymers of hydroxybutyric acid and hydroxyvaleric acid, polyesters of succinic acid, polylactic acid, cross-linked hyaluronic acid, poly(organo)phosphazanes, biodegradable polyurethanes, and PGA cross-linked to collagen. These spiral conduits may also include a layer of polymeric hydrogel adhering to the layer of cells on the support or to the support itself. The hydrogels disclosed as employable include fibrin glues, Pluronics®, polyethylene glycol (PEG) hydrogels, agarose gels, PolyHEMA (poly 2-hydroxyethylmethacrylate) hydrogels, PHPMA (poly N-(2-hydroxypropyl) methacrylamide) hydrogels, collagen gels, Matrigel®, chitosan gels, gel mixtures such as mixtures of collagen, laminin and fibronectin, alginate gels and collagen-glycosaminoglycan gels.

In tissue culture research, it has been proposed to employ self-assembled monolayers (SAMs) to culture cells. See for example United States Patent Publication 2004/0023414 in the name of Zhang et al. In these SAMs, an array of isolated regions on a solid support created by a preselected, reproducibly patterned, layer of amino-terminally bound peptides are disclosed as employable in culturing cells. The cells can be attached to the SAMs and maintained under suitable growth conditions in order to study cell growth and cellular interactions to external stimuli such as other cells, growth factors, repellants and inhibitors, to assist in the manufacture of cellular products like proteins hormones, and to assist in the manufacture of artificial tissues such as fibroblasts, endothelial cells, smooth muscle cells and neuronal cells for tissue graft purposes. The SAM polymer employed is polydimethylsiloxane (PDMS). The general technique for fabricating complex surface topographies in organic polymers (SAMs) by replica molding against an elastomeric master is disclosed in United States Patent No. 5,512,131, in greater detail in United States Patent No. 6,180,239 and in Xia et al., Science 273 (1996) 347-49. The '239 patent discloses that a surface such as a silicon dioxide or glass slide or Petri dish can be patterned with a SAM of alkylsiloxane that exposes a chemical functionality of biological interest.

United States Patent Publication 2004/0121066 in the name of Anderson et al. discloses, in addition to the preferred polymer PDMS, a wide variety of polymeric materials that can be employed to create structures with preselected, patterned surfaces, including polyurethanes, polyamides, polycarbonates, polyacetylenes, and polydiacetylenes, polyphosphazenes, polysiloxanes, polyolefins, polyesters, polyethers, poly(ether ketones), poly(alkaline oxides), poly(ethylene terephthalate), poly(methyl methacrylate), polystyrene and derivatives, and block, random, radial, linear or teleblock copolymers thereof. The patterned surfaces can be employed to deposit a patterned layer of cells on the surface, for in vitro assays, to study angiogenesis during tumor formation and to study molecular interactions between different cell types that underlie embryonic morphogenesis.

United States Patent Publication 2004/0020774 in the name of Aksay et al. discloses, in broad outline, the application of the SAMs technique to form polycrystalline inorganic substrates using a network of patterned capillaries formed by placing an elastomeric stamp made of PDMS possessing designed relief features on the surface of the substrate.

Additional work has been directed to the use of randomly textured interior surfaces created by "demixing" of polymers such as polystyrene/poly(4-bromostyrene) and polystyrene/poly(n-butylmethacrylate) deposited in nylon tubing. Fibroblasts seeded in these structures and fibroblasts seeded in control tubes having smooth interior surfaces displayed markedly different morphology, size and adhesion characteristics. Berry et al., Biomaterials 26 (2005) 4985-92.

Researchers also have characterized the growth of human connective tissue progenitor cells (CTPs) on smooth and textured channels composed of PDMS polymeric substrates and have found that the cells on smooth PDMS substrates and on control surfaces spread and proliferated as colonies in proximity to other cells and migrated in random directions covering significantly larger areas than colonies formed on channel textured PDMS substrates having curved cross-sections 11 µm high, 45 µm wide and separated by 5 µm ridges. The cells grown on channel textured PDMS substrates proliferated and spread in a highly directional manner, aligning and migrating preferentially along the channel axis and they created colonies that were more dense and with significantly longer axes. See for example, Mata et al., Biomedical Microdevices 4 (2002) 267-75.

Implantation of biodegradable, biocompatible synthetic polymeric fibers seeded with tenocytes (cells obtained from tendons) and ligamentum cells (cells obtained from ligaments) has been attempted. See United States Patent Publication 2005/0060033 in the name of Vacanti et al. Embossed non-woven polyglycolic acid mesh, 100 microns thick with interstitial spacing of random or parallel fibers between 75 and 100 microns was employed. The specimens created from implantation of tenocytes onto polymer fibers arranged in parallel showed, at six weeks, a greater degree of parallel collagen fibril organization than those on polymer fibers arranged randomly, but at ten weeks the fibers were arranged with proper anatomic cellular organization regardless of polymer fiber orientation.

Guidance of neurite outgrowth has been demonstrated *in vitro* with culture substrates that contain micropatterned features on the nanometer to micron scale. For example, see Mata et al., supra. However, it has recently been reported that neurite extension occurs *across* micropatterned grooves having feature sizes on the order of tens of microns, sizes relevant to the design of biomaterials and tissue engineering scaffolds known in the art. Goldner et al., Biomaterials 27 (2006) 460-472. Goldner demonstrates that to be useful in inducing and stimulating guided cell growth, substrates of the prior art type having grooves and ridges must be carefully designed and constructed if cell growth is intended to be confined to the grooves. For example, if the depth of the groove is 50 µm, a groove width on the order of at least 110 µm and a plateau width on the order of at least 200 µm is necessary to constrain neurite extension. Thus, in light of these results the prior art substrates employed to guide the growth of neurites may not work as anticipated in some cases and under certain conditions. A new approach might be advantageous.

### SUMMARY OF THE INVENTION

The invention is based on the premise that employing substrates having cell-templated surface features that impart the surface geometry, i.e., the morphology, of the cell to the substrate results in increased cellular adhesion and integration into the cellular environment when the substrate is employed as an implant to induce or stimulate cell and tissue growth, differentiation and regeneration. Thus, the cells are employed as templates to impart the morphology of the cell to the substrate. The substrate, now incorporating the external morphology of the cell template, can then be employed to control the growth and differentiation of cells in vitro or in vivo. Prior work in the field, some of which is discussed above, suggests that substrates with nanometer-scale and micrometer-scale surface features can promote cell adhesion in contrast to substrates with smooth surfaces. The prior work has focused on producing substrates having arbitrarily designed three-dimensional surface features composed, for example, of regularly-spaced ridges and troughs. However, neurite bridging across the troughs of such substrates has been observed, suggesting that regularly-spaced ridges and troughs may not be the ideal design for such structure. See Goldner, et al., supra. To our knowledge, the use of cells to create the three-dimensional surface features or morphologies in substrates, including in implantable substrates, has not heretofore been suggested or disclosed.

In one aspect, the invention comprises a substrate for influencing the organization, spreading or adhesion of a selected cell to induce or stimulate motility, growth, differentiation or regeneration of the cell or of tissue constituting the cells. The substrate comprises a non-toxic, biocompatible film having a cell-templated morphology that substantially reproduces the cellular morphology of the selected cell such that growth, differentiation or regeneration of the selected cell is induced or stimulated in the presence of the substrate. The film can be in the shape of a planar or substantially planar sheet having a relatively high length to width and length to thickness ratio. Alternatively the film can be formed into a tube, a channel or a conduit or a thick film may be formed into a rectangular bridge. Whichever structure chosen for the substrate the substrate is formed with a surface from which the morphology of the templated cell projects and that substantially reproduces the topographic morphological features of the selected cell. Thus, if the film is formed into a planar sheet or a thicker rectangular bridge, one side or surface of the device will exhibit the surface morphological features of the cell template as depressions in the surface of the film. A positive "image" of the cell can be obtained by using the film as a "negative substrate" and curing a second film placed on top of the feature bearing side of the first film "negative". If the film is formed into a conduit, the conduit will have an interior surface from which the morphology of the templated cell projects either exteriorly or interiorly to create a patterned interior or exterior surface that substantially reproduces the native growth and differentiation pattern of the selected cell.

Preferably, the substrate is a biocompatible implantable device. Exemplary is a nerve growth guidance device. The implant may be bioerodible or may be permanent, depending upon the application. For example, a bioerodible implant may be employed when transplanted cells are expected to produce their own extracellular matrix over time as the material degrades, thus replacing the material with natural tissue. And alternatively, a permanent implant may be employed when transplanted cells are expected to require material support for the lifetime of the implant.

Any cell type may be employed as the template. Exemplary are neuronal cells, glial cells (Schwann cells, astrocytes, oligodendrocytes), connective tissue cells (fibroblasts, osteoblasts), muscle cells (smooth, skeletal, cardiac), endothelial cells, or a stem cells. Preferred are neuronal cells and muscle cells. The cells may be employed alone or with their extracellular matrix. For example, polymer films whose surfaces incorporate topographical features of aligned Schwann cells may be made. The topographical features will include indented and protruding cellular feature of the cells. The film may be cut to the appropriate size and employed or it may be fashioned into a nerve growth guidance device, as described below.

The non-toxic, biocompatible substrate may be formed from and composed of an elastomeric, natural or synthetic, polymeric gel or solid polymer. The polymeric gel or solid should be a curable polymer that is flowable in liquid phase, capable of conversion to a rubbery or gelled solid upon curing, detachable from a cell template, and capable of maintaining the cell-templated dimensions and geometry upon detachment from the cell template. Exemplary elastomeric polymeric gels or solids that can be employed include alkylsiloxanes, polylactic acids, poly(D,L-lactides), copolymers of lactic acid and glycolic acid, copolymers of lactic acid and e-aminocaproic acid, polyhydroxylakanoates, polyesters, polyglycolic acids, polycaprolactones, polydesoxazons, copolymers of hydroxybutyric acid and hydroxyvaleric acid, cross-linked hyaluronic acid, poly(organo)phosphazine, biodegradable polyurethanes, polyorthoesters, polyglycolic acid cross-linked to collagen, copolymers of collagen and a glycosaminoglycan, copolymers of L-lactide and ε-caprolactone, mixtures of polyurethanes and polylactic acid, mixture of a polyimide and a polystyrene, a cross-linked hyaluronic acid, poly(organo) phosphazanes, biodegradable polyurethanes, fibrin glues, Pluronics®, polyethylene glycol (PEG) hydrogels, agarose gels, poly 2-hydroxyethylmethacrylate hydrogels, poly N-(2-hydroxypropyl) methacrylamide hydrogels, collagen gels, Matrigel®, chitosan gels, and gel mixtures such as a mixture of collagen, laminin and fibronectin, an alginate gel and a collagen-glycosaminoglycan gel. Alkysiloxanes are preferred and especially preferred is polydimethyl-siloxane (PDMS) or poly(ethoxymethylsiloxane). Copolymers of lactic acid and glycolic acid are also preferred.

In some embodiments, it may be desirable to include bioactive molecules within or on the implantable substrate. Such bioactive molecules can assist in the inducement of growth, repair and regeneration. Exemplary bioactives include growth factors, glycoproteins of the ECM, neuropoietins and neurotrophins, for example nerve growth factor (NGF), ciliary neurotrophic factor (CNTF), glial cell line-derived neurotrophic factor (GDNF), neurotrophin 4/5 (NT4/5), motor nerve growth factor, (MNGF), brain derived neurotrophic factor (BDNF), FK506, heat shock protein 27 (HSP 27), insulin-like growth factor (IGF-1), insulin-like growth factor 2 (IGF-2), the platelet derived growth factors, glial growth factor (GGF), interleukin-1 (IL-1), acidic and basic fibroblast growth factors, epidermal growth factor (EGF), vascular endothelium growth factor (VEGF), muscle morphogenic factor (MMF), TGFβ, TGFa, LIF, 4-methylcatechol, tacrolimus, inosine, spermine, spermidine, laminin, collagen and polylysine. With respect to the repair and regeneration of neural tissue Schwann cells may be included as a guidance factor or cue to stimulate neuronal growth and differentiation; additional bioactive molecules or nerve growth guidance cues that may be employed in making and using a nerve growth guidance device of the invention can be found in Schmidt and Leach, Annu. Rev. Biomed. Engin. 5 (2003) 293-347. In the nerve growth guidance device of the invention, the cell-templated, polymeric film is formed in the shape of a conduit and one or more nerve growth guidance cues is optionally deposited inside the conduit or is included within the film.

The nerve growth guidance devices are formed from films templated with neural cells, such as Schwann cells or astrocytes, and they bear the surface topography and features that impart the surface geometry, i.e., the morphology, of such cells. The cell-templated conduit may comprise a bioerodable implant having an interior surface from which the morphology of the templated cell projects either exteriorly or interiorly to create a patterned interior or exterior surface that substantially reproduces the native growth and differentiation pattern of the selected cell.

In another aspect, the invention comprises a method of patterning the surface of an elastomeric polymer film substrate with dimensions and geometries that substantially reproduce the dimensions and geometries of a selected cell. The method comprises the steps of forming a pre-polymer solution from the starting composition, placing in contact with the pre-polymer solution a cell template composed of the selected cells, allowing the pre-polymer to cure and removing the substrate from the cell template. Optionally, the method may also include the step of adding a bioactive molecule or guidance cue to the pre-polymer solution in order to assist in the growth and regeneration promotion. Alternatively, the bioactive molecule or guidance cue may be coated onto the cell-templated substrate after curing and removal of the substrate from the cell template. Molecules or cues that may be included or employed as coatings are set forth above.

The selected cell may be a neuronal cell, a glial cell (a Schwann cell, an astrocyte, or an oligodendrocyte for example), a connective tissue cell (a fibroblast cell, a myofibroblast cell or an osteoblast cell for example), a muscle cell (a smooth muscle cell, a skeletal muscle cell, or a cardiac muscle cell for example) an endothelial cell, or a stem cell, provided the stem cell is not provided from a human embryo.

The pre-polymer used to form the solution should be a curable elastomeric, natural or synthetic, polymeric gel or solid that is flowable in liquid phase, capable of conversion to a rubbery or gelled solid upon curing, detachable from a cell template, and capable of maintaining the cell-templated dimensions and geometry upon detachment from the cell template. Exemplary pre-polymers include alkylsiloxanes, polylactic acids, poly(D,L-lactides), copolymers of lactic acid and glycolic acid, copolymers of lactic acid and ε-aminocaproic acid, polyhydroxylakanoates, polyesters, polyglycolic acids, polycaprolactones, polydesoxazons, copolymers of hydroxybutyric acid and hydroxyvaleric acid, cross-linked hyaluronic acid, poly(organo)phosphazines, biodegradable polyurethanes, polyorthoesters, a polyglycolic acid cross-linked to a collagen, copolymers of a collagen and a glycosaminoglycan, copolymers of L-lactide and ε-caprolactone, mixtures of polyurethane and polylactic acid, mixtures of a polyimide and a polystyrene, poly(organo) phosphazanes, fibrin glues, Pluronics®, polyethylene glycol (PEG) hydrogels, agarose gels, poly 2-hydroxyethylmethacrylate hydrogels, poly N-(2-hydroxypropyl) methacrylamide hydrogels, collagen gels, Matrigel®, chitosan gels, and gel mixtures such as a mixture of collagen, laminin and fibronectin, alginate gels and a collagen-glycosaminoglycan gel. Additional exemplary pre-polymers are set forth in the detailed description. Preferred are polydimethylsiloxane, poly(ethoxymethylsiloxane) and copolymers of lactic acid and glycolic acid.

In a modification of the method once the ideal cell-templating dimensions are established they can be reproduced (with or without modification) using a computer-assisted design program such as AutoCAD®.

In another aspect, the invention includes a method of repairing injured cells or tissues of a selected cell type in a mammalian patient. The method comprises providing a cell-templated substrate having dimensions and geometries that substantially reproduce the dimensions and geometries of the selected cell type; positioning the cell-templated substrate in proximity to the injured cells or tissues; and allowing new cells or tissues of the selected type to grow onto the cell-templated substrate. The cell-templated substrate may optionally include or be coated with one or more bioactive molecules or guidance cues in order to assist in growth and differentiation of new cells and tissues. Molecules or cues, for example laminin to promote neurite extension or collagen to promote fibroblast adhesion, may be included or employed as coatings are set forth above.

The selected cell may be a neuronal cell, a glial cell (a Schwann cell, an astrocyte, or an oligodendrocyte for example), a connective tissue cell (a fibroblast cell, a myofibroblast cell or an osteoblast cell for example), a muscle cell (a smooth muscle cell, a skeletal muscle cell, or a cardiac muscle cell for example) an endothelial cell, or a stem cell, provided the stem cell is not provided from a human embryo. The cells may be employed alone or with their extracellular matrix.

The cell-templated substrate may comprise a bioerodable implant and the implant may be a conduit formed in the shape of tube or a channel with an interior surface from which the morphology of the templated cell projects either exteriorly or interiorly to create a patterned interior tube or channel surface that substantially reproduces the native growth and differentiation pattern of the selected cell. Exemplary substrates for use in the method include those set forth above and in the detailed description. Preferred substrates are polydimethyl-siloxane, poly(ethoxymethylsiloxane) and copolymers of lactic acid and glycolic acid.

In a specific embodiment, the method comprises a method for regenerating a severed or damaged nerve. In this embodiment the method comprises the steps of providing a cell-templated conduit with an interior surface from which the morphology of neuronal cells or glial cells projects either exteriorly or interiorly to create a patterned interior tube or channel surface that substantially reproduces the native growth and differentiation pattern of the neuronal or glial cells, positioning the cell-templated conduit in proximity to an end of the severed or damaged nerve and allowing the severed or damaged nerve to grow into the conduit. The interior of the cell-templated conduit may be coated with one or more nerve growth guidance cues to assist in stimulating the regeneration of the severed nerve endings. Preferred cues include laminin and nerve growth factor (NGF). The conduit may be sutured into place using methods known in the art.

### DESCRIPTION OF THE DRAWINGS

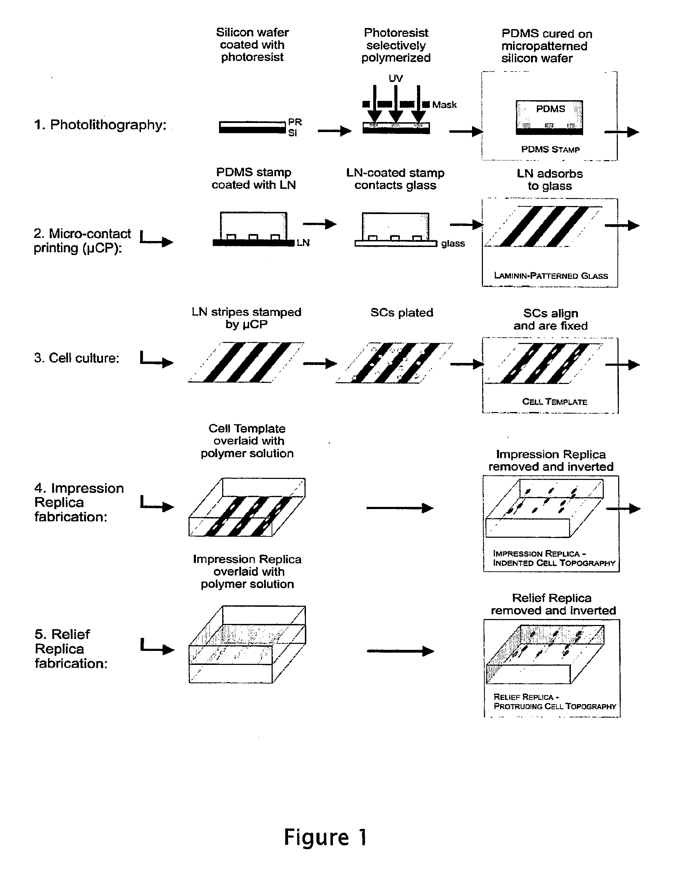

Figure 1 is an illustration of the method of making the cell-templated structures of the invention, highlighting the 5 main steps: (1) Production of a polymeric stamp for micro-contact printing; (2) micro-contact printing of laminin stripes onto glass coverslips; (3) Cell culture; (4) Fabrication of the impression replica with indented topographical features; and (5) Fabrication of the relief replica with protruding topographical features. The method can begin with step 4 using fixed cells, if no alignment of cells is desired.

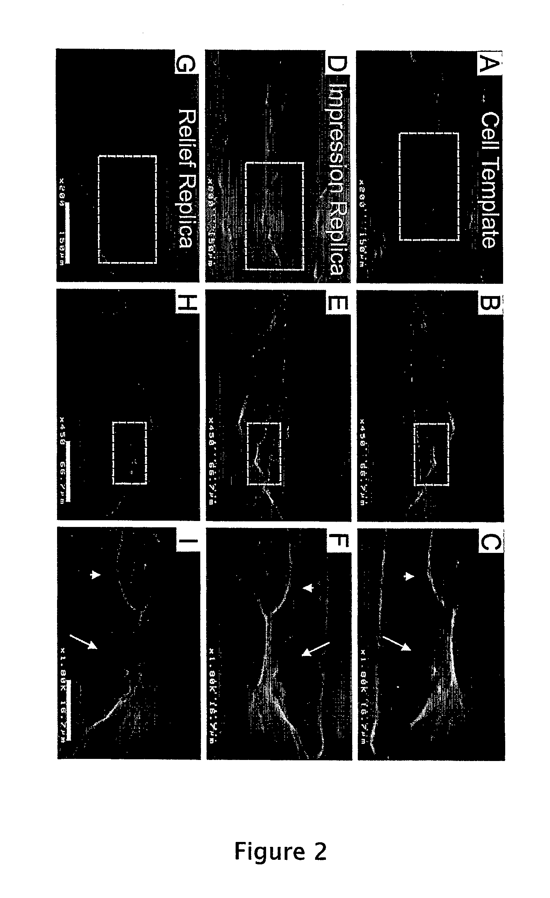

Figure 2 is a xerographic reproduction of a series of SEM micrographs of a cell-templated film, templated with aligned Schwann cells, showing the replicated cellular features. Corresponding regions of the impression replica and the relief replica show replication of the cellular topographical features in the original template. A-C are reproductions of SEM micrographs of the SC template; D-F are reproductions of the SEM micrographs of the impression replica and G-I are reproductions of the SEM micrographs of the relief replica. Rectangle outlines the borders of the region that is magnified in the image to the right. Note that inherent to the process, features in the impression replica are inverted relative to those of the template and the relief replica. Scale bars: 2G = 150 µm, 2H == 66.7 µm, 2I = 16.7 µm.

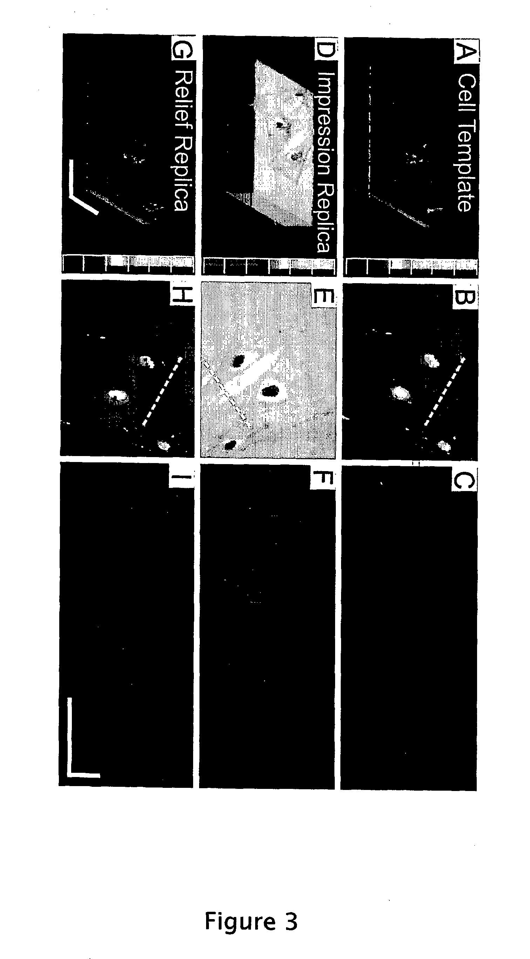

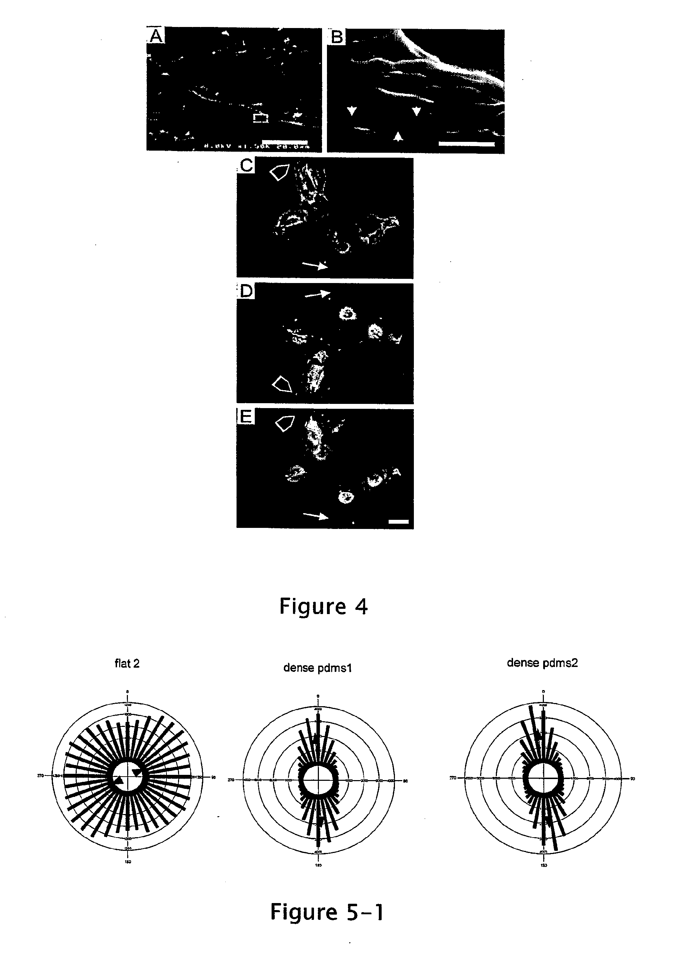

Figure 3 is a xerographic reproduction of the quantification, by WIM (White-light Interference Microscopy) of the aligned Schwann cells shown in Figure 2. Corresponding regions of the impression replica (D-F) and the relief replica (G-I) replicate the sizes and shapes of cellular topographical features in the original template (A-C). Oblique images (A, D, G) provide a three-dimensional view of each substrate; corresponding overhead images (B, E, H) provide a top view of each surface; and corresponding surface profile plots (C, F, I) provide measurements of a cross-section along the line superimposed on each overhead image. Color bars in A, D, and G show pseudocolor scales where the range of each entire color bar represents a z-axis difference of 2.25 µm (A), 2.55µm (D), and 2.03µm (G). Scale bars in G: x-axis = 50µm, y-axis = 70µm; scale bars in I: x-axis = 20µm, z-axis = 20nm. Figure 4 are xerographic reproductions of SEM micrographs of Schwann cell templated films and rat aortic smooth muscle cell (SMC) templated films made as described in Example 2.
Figure 4A and 4B are photographic reproductions of polyurethane film impression replicas containing indented topographical features of aligned SC. This region corresponds to cellular extensions (white arrowheads). The rectangle in A outlines the region that is magnified in B, in which nanoscale features are visible. Figure 4C-4E photographic reproductions of PDMS film impressions and relief replicas containing indented and protruding topographical features of SMC. Phase-contrast micrographs of corresponding regions of the impression replica (D), and the relief replica (E) show replication of cellular features in the original SMC template (C). Arrows, lamellipodia; black arrowheads, filopodia. Scale bars, 4A = 20µm, 4B = 1µm, 4E = 50µm.

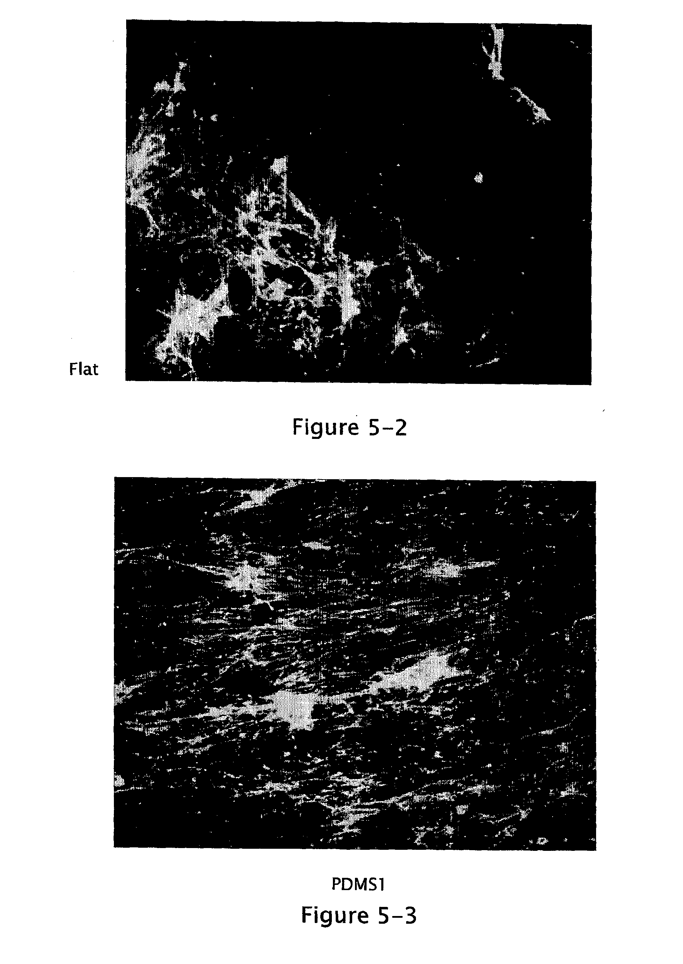

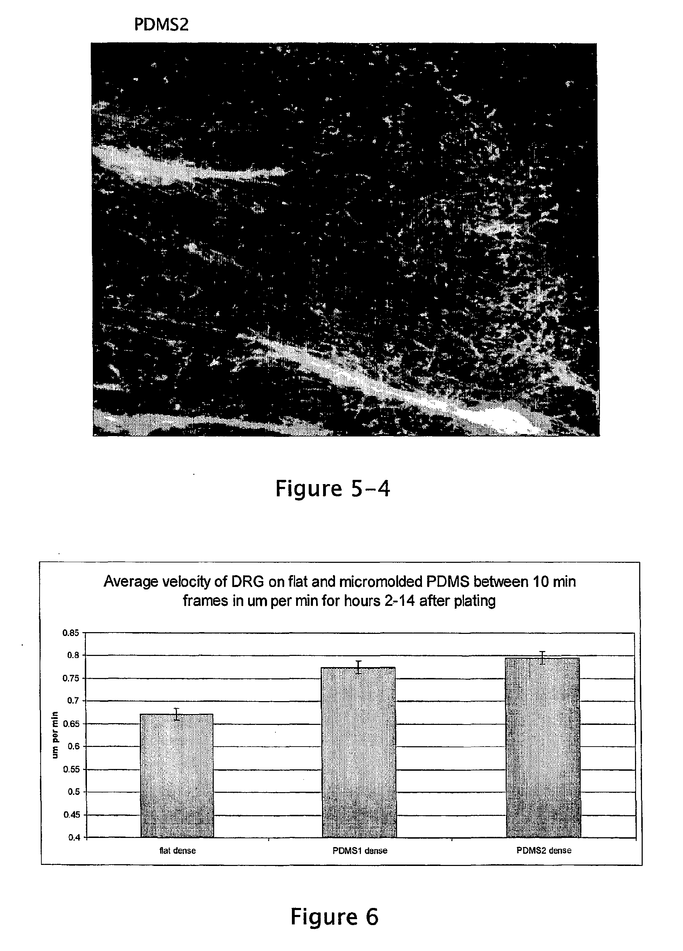

Figure 5 is a graphic illustration of the results of the experiment described in Example 3, part (i). The photographic reproductions (Fig. 5-2, 5-3, and 5-4) show the cells templated on flat, PDMS1 and PDMS2 respectively. The illustration (Fig. 5-1) shows the results processed in ImageJ and averaged as explained in detail in the Example.
Figure 6 is a graphic illustration and xerographic reproductions of photographic images taken of the results of the experiment described in Example 3, part (ii).

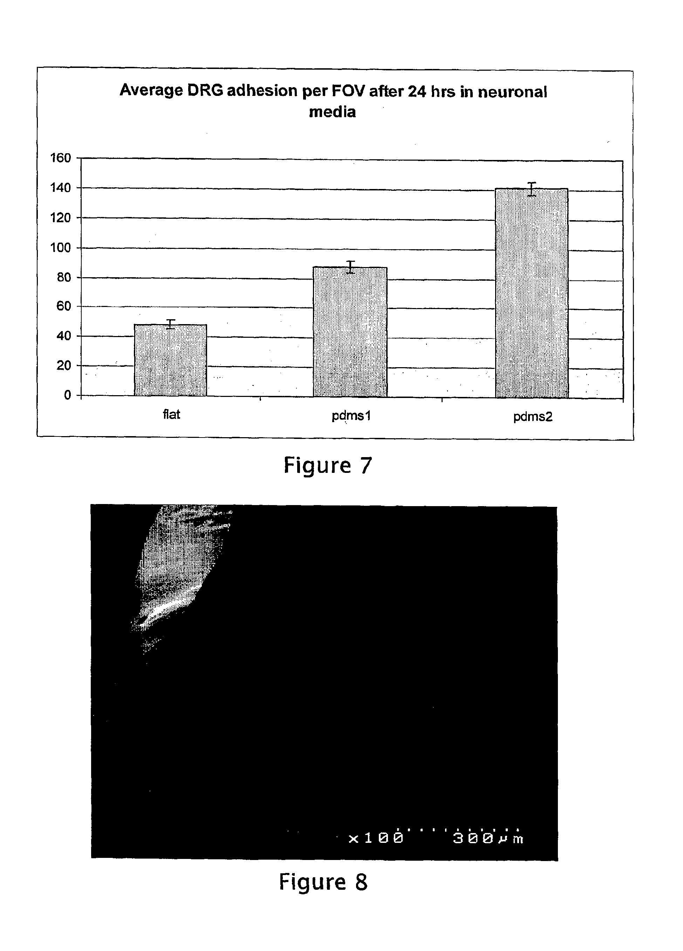

Figure 7 is a graphic illustration of the results of the experiment described in Example 3, part (iii).
Figure 8 is a photographic reproduction of a SEM micrograph at 100x magnification of a nerve growth guidance device made in accordance with the invention, with indented, radially oriented SC-shaped indentations on the luminal side. The tube consists of two successive layers of PDMS1 film, each ∼30um thick, rolled around a glass mandrel with 1mm diameter.

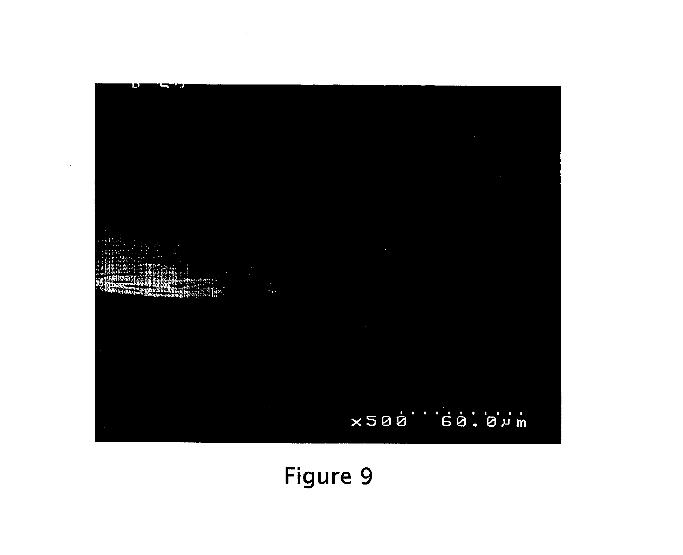

Figure 9 is a detail from Figure 8, showing SC-shaped indentations and two distinct layers of PDMS1 film at 500x magnification.

### DETAILED DESCRIPTION OF THE INVENTION

### Definitions

The following words and phrases employed in this specification have the meanings set forth below.

"Biocompatible" means compatible with a living system or entity and not detrimental to the general existence and functioning of the system or entity; e.g., neither toxic to, nor causing a detrimental reaction (like an immunological reaction) in a living system such that it would make it undesirable to continue its use.

"Bioerodible" means susceptible to degradation over time, usually months.

"Biomaterial" is a synthetic, semi-synthetic or natural material used in a biological system in an implantable manner. A biomaterial is chosen for it biocompatibility.

"Curable" refers to the ability to change the properties of a plastic or resin by chemical reaction, for example by condensation, polymerization, or addition. The reaction may be accomplished by the action of heat with or without pressure.

"Cellular morphology" refers to the form, especially the external form, of a cell. It encompasses the external dimensions of the cell as well as the topographies and geometries of the exterior of the cell membrane.

"Elastomer" is a natural or synthetic material having elastic properties similar to rubber.

"Film" is a very thin, sheet-like or membrane-like material or structure. The thickness dimension of the films of this invention will depend on the selected cell type chosen as the template, the morphology of that cell type and the elastomeric characteristics of the substrate employed. The skilled artisan will be able to choose an appropriate thickness for the substrate employed without undue experimentation. A suggested range from a minimum of about 15 µm to a maximum of about 2 cm is believed appropriate. For ease of use, a preferably range is believed to be from about 3-5 mm. If a thinner film is desired for a particular use, a, preferable range is believed to be from about 20 µm to about 50 µm.

"Gel" is colloidal suspension of a liquid in a solid forming a jelly-like material in a solid form. "Hydrogel" is a water-based gel.

"Nerve growth guidance device" means a structure that is formed from a composition that can be used in a living system or entity to induce, enhance, stimulate or control the growth of neuronal cells and tissues.

"Nerve growth guidance cue" is a chemical entity that is able to induce, enhance, stimulate or control the growth of neurons. Any biological molecule that stimulates, induces, enhances or controls the growth of neurons, either by direct or indirect activation or by repression or inhibition of nerve growth inhibitor substances may be considered a nerve growth guidance cue.

"Polymer" is a large molecule formed by union of at least five identical monomers. It may be natural or synthetic. A "polymeric material" is a material formed from one or more polymers.

"Pre-polymer" is a polymer of relatively low molecular weight, usually intermediate between that of the monomer and the final polymer or resin, which may be mixed with compounding additives, and which is capable of being hardened by further polymerization during or after a forming process.

### Description of the Invention

The invention is based on the premise that employing substrates having cell-templated surface features that impart the surface geometry, i.e., the morphology, of the cell to the substrate results in increased cellular adhesion when the substrate is employed as an implant to induce or stimulate cell and tissue growth, differentiation and regeneration. Thus, the cells are employed as templates to impart the morphology of the cell to the substrate. The substrate, now incorporating the external morphology of the cell template, can then be employed to control the growth and differentiation of cells in vitro or in vivo.

The substrates can be employed for influencing the organization, spreading or adhesion of a selected cell to induce or stimulate growth, differentiation or regeneration of the cell or of tissue constituting the cells. The substrates can also be employed to facilitate phase or DIC imaging of non-transparent surfaces, in image subtraction techniques to distinguish topography versus cell-interior elements, and to produce replica molds of ocular lenses (for ocular implants for example). The substrates can be used to enhance adhesion of cell receptors and may find use in biological assays, to present antigens or epitopes to the immune system, and to pattern the transdermal portion of limb replacements to foster skin attachment. Additional uses include in semipermeable membrane patterning to promote adhesion or orientations of living cells in bioreactor designs, for example in the manufacture of artificial kidneys and other organs without having to apply shear flow techniques, in skin graft culture methods to provide molds of the dermal-epidermal interface, to provide precise volumetric measurements of complicated tissue shapes, for guiding in-growth and promoting bone implant adhesion, to reduce platelet activation in vascular grafting, in the preservation or archaeological artifacts, and in the encapsulation of actual cell or microbe materials to provide for safe handling, for example for teaching samples, transport, storage, and viewing, even by untrained staff. For example, one could send replicas of tissue samples from patients through the mail and store them indefinitely, provide replicas of ebola-infected tissues to teaching laboratories and medical schools or provide replicas of tuberculosis bacteria to high school classrooms. All of these replicas could be handleable and washable.

The substrate comprises a non-toxic, biocompatible film having a cell-templated morphology that substantially reproduces the cellular morphology of the selected cell such that growth, differentiation or regeneration of the selected cells or tissues is induced or stimulated in the presence of the substrate.
Preferably, the substrate comprises a bioerodable film that can be implanted into a mammalian patient. Preferably the patient is a human patient. However, the patient could be a veterinary patient, for example, an equine, canine or feline patient.

In this aspect, the implantable film could be formed in the shape of tube or a channel with an interior surface from which the morphology of the templated cell projects exteriorly to create a patterned interior tube or channel surface that substantially reproduces the native growth and differentiation pattern of the selected cell. Alternatively, the film could be formed into a rectangular band-like structure having relatively high length to thickness and length to width ratios. In this aspect, both top and bottom surfaces of the band would be templated. The film could be a substantially planar band-like film having an arcuate longitudinal depression. A band-like film optionally could be formed with internal or external flanges. Alternatively, the tube or channel could have an interior surface from which the morphology of the templated cell projects interiorly. In this latter aspect, a hollow interior surface is unnecessary; a rod, column, or rope-like structure could be employed.

In nerve regeneration and repair applications, the dimensions of the implantable substrate film should be substantially equivalent to those of the injury gap being bridged, for example in the case of a spinal cord injury, or the peripheral nerve being replaced. Thus, the implants of the invention are implanted in the same manner as other reconstructed tissues, ligaments, nerves, tendons and the like. They can be surgically interposed between the cut ends and sutured in place with biodegradable suture material. Such procedures art known in the art and described for example in Hadlock et al., Archives of Otolaryngology-Head & Neck Surgery 124 (1998) 1081-86; Wang et al., Microsurgery 14 (1993) 608-18 and Mackinnon et al., Plast. Reconstr. Surg 85 (1990) 419-24. Additional techniques that may be employed are disclosed in Schmidt and Leach, Annu. Rev. Biomed. Eng. 5 (2003) 293-347; United States Patent No. 6,676,675; and United States Patent Publication Nos. 2002/0051806 and 2001/0031974.

Films templated with fibroblasts and/or smooth muscle cells could be used to promote adhesion of endothelial cells for vascular grafts in the same manner as that described for non-templated randomly textured or patterned films as is described in Miller et al. J Biomed Mater Res 73A (2005) 476-484. Films templated with fibroblasts could also be used to promote adhesion of smooth muscle cells for tissue engineered bladder in the same manner as that described for non-templated randomly textured or patterned films as is described in Thapa et al. Biomaterials 24 (2003) 2915-2926.

Preferably, the implantable film in the desired shape is formed from an elastomeric, natural or synthetic, polymeric gel or solid. The elastomeric polymeric gel or solid may be formed from a curable polymer that is flowable in liquid phase and capable of conversion to a rubbery or gelled solid upon curing. It should be detachable from the cell template and capable of maintaining the cell-templated dimensions and geometry upon detachment. Suitable polymeric materials may have linear or branched backbones and may be crosslinked or non-crosslinked depending upon the particular polymer and the degree of formability desired. Examples of suitable polymeric materials include the general classes of silicone polymers, epoxy polymers and acrylate polymers. Examples of silicone polymerics suitable for use include those formed from chlorosilane precursors such as methyl chlorosilanes, ethyl chlorosilanes and phenyl chlorosilanes. Preferred are alkylsiloxane and especially preferred is polydimethylsiloxane (PDMS), which is sold under the trademark Sylgard® by Dow Chemical Co., Midland, MI. and poly(ethoxymethylsiloxane). Also preferred are copolymers of lactic acid and glycolic acid. Epoxy polymers have a three-member cyclic ether group (an epoxy group, a 1,2-epoxide or an oxirane). Examples of epoxy polymerics include diglycidyl ethers of bisphenol A and the Novolac polymers. Acrylate polymers are copolymers of acrylic acid, methacrylic acids and esters of these acids or acrylonitrile.

Other polymeric materials that can be used include polylactic acids, poly(D,L-lactides), copolymers of lactic acid and glycolic acid, copolymers of lactic acid and ε-aminocaproic acid, polyhydroxylakanoates, polyesters, polyglycolic acids, polycaprolactones, polydesoxazons, copolymers of hydroxybutyric acid and hydroxyvaleric acid, cross-linked hyaluronic acids, poly(organo)phosphazines, biodegradable polyurethanes, polyorthoesters, polyglycolic acids cross-linked to collagen, copolymers of collagen and a glycosaminoglycans, copolymers of L-lactide and ε-caprolactone, mixtures of polyurethanes and polylactic acids, mixture of polyimides and polystyrenes, fibrin glues, Pluronics®, polyethylene glycol (PEG) hydrogels, agarose gels, poly 2-hydroxyethylmethacrylate hydrogels, poly N-(2-hydroxypropyl) methacrylamide hydrogels, collagen gels, Matrigel®, chitosan gels, and gel mixtures such as mixtures of collagen, laminin and fibronectin, alginate gels and collagen-glycosaminoglycan gels.

Any cell type is employable as a template. Preferred cells employable as templates include neuronal cells, glial cells, for example Schwann cells (SC), astrocytes or oligodendrocytes, connective tissue cells, such as fibroblasts, myofibroblasts and osteoblasts, muscle cells such as smooth muscle (SMC), skeletal muscle and cardiac muscle cells, endothelial cells and stem cells provided the stem cell is not provided from a human embryo. The cells may be employed alone or in conjunction with their ECM. Cancer cells may be employed as templates or may be encapsulated. In addition, tissues, for example ocular tissue and skin tissue, and microorganisms, for example bacteria and viruses, may be employed as templates in some of the uses discussed infra.

In some embodiments it may be desirable to include bioactive molecules or guidance cues with the implantable templated films of the invention. A variety of bioactive molecules can be delivered in order to assist in the inducement of growth, repair and regeneration. They can either be coated onto the substrate after templating or included in the pre-polymer. Examples of bioactive agents having neurotrophic activities which are employable as nerve growth guidance cues include nerve growth factor (NGF), ciliary neurotrophic factor (CNTF), glial cell line-derived neurotrophic factor (GDNF), ephexin 1, ephrin, neurotrophin 4/5 (NT4/5), motor nerve growth factor, (MNGF), brain derived neurotrophic factor (BDNF), heat shock protein 27 (HSP 27), insulin-like growth factor (IGF-1), insulin-like growth factor 2 (IGF-2), the platelet derived growth factors, glial growth factor (GGF), interleukin-1 (IL-1), acidic and basic fibroblast growth factors, 4-methylcatechol, tacrolimus, inosine, spermine, spermidine, laminin, collagen and polylysine. With respect to nerve regeneration, additional bioactive molecules and guidance cues, especially nerve guidance cues, are disclosed in Schmidt and Leach, Annu. Rev. Biomed. Eng. 5 (2003) 293-347.

### Examples

All coating and cell culture reagents are from Invitrogen Life Technologies unless otherwise indicated.

Cells are cultured as detailed below for each cell type. All cultures are incubated at 37°C and 5% CO₂ in a humidified environment. For culture on micropatterned substrates, cells are initially plated at specified densities onto substrates in 100µl of appropriate plating medium. After cells adhere to substrates for 3 h, 3 ml of appropriate culture media are added.

PDMS substrates are plasma activated at 10.5 W for 60 sec with a plasma cleaner/sterilizer (PDC - 32 G, Med RF level, Harrick), sterilized by immersion in 70% ethanol, and rinsed with sterile dH₂O. Substrates are coated with 100 µg/ml poly-L-lysine (PLL, 70-150 kDa, Sigma) for one hour, rinsed with dH₂O, coated with 50 µg/ml mouse laminin in Hanks' Balanced Salt Solution without calcium or magnesium (HBSS-CMF) for one hour, and rinsed with dH₂O before plating.

### Example 1. Preparation of cell-templated substrates

### (i)Template cell culture alignment

Aligned cell cultures, for example Schwann cell cultures, are prepared by micro-stamping laminin in a striped pattern onto a glass coverslip, then culturing Schwann cells on the micropatterned coverslip. For micro-stamping, a grooved PDMS stamp is prepared as described in Goldner et al. (2005). Briefly, the pattern is designed in AutoCAD LT 2004 (Autodesk Corp.) and printed at 10,000 dpi onto a mylar mask. Standard photolithographic techniques are used to transfer the pattern onto Si wafers (Silicon Sense, Inc.) spin-coated with a 50 µm layer of negative tone Nano SU-8 50 photoresist (Microtech Corp.), resulting in repetitive grooves with 60 µm groove, width, 60 µm plateau width, and 50 µm groove depth. Finished features are silaned to prevent adhesion during subsequent casting.

### (ii) Polymer stamp manufacture

Sylgard 184 PDMS Elastomer base is mixed with Sylgard 184 PDSM curing agent at a 10:1 wt/wt ratio, degassed, poured onto a micro-patterned wafer to a thickness of 1-2 mm, cured at 95°C for 45 min, and peeled off the wafers to generate substrates of 1 cm x 1 cm x 1-2 mm for micro-contact printing. The stamps are then submerged in 10% sodium dodecyl sulfate (SDS, Sigma) in dH₂O, washed, and incubated with 50 µg/ml mouse laminin in Hanks' balanced salt solution without calcium or magnesium (HBSS-CMF) for 1 h. Stamps are inverted and left overnight in contact with glass coverslips that had previously been plasma activated (as described above) with a plasma cleaner/sterilizer (PDC-32G, Med RF lever, Harrick) to promote protein transfer. After the stamps are removed, the substrates were gently washed with HBSS-CMF to remove excess unbound laminin.

### (iii) Schwann cell templating

Schwann cells are isolated from adult rat sciatic nerve as described in Goldner et al. Briefly, a section of sciatic nerve from an adult rat is harvested, cleaned of epineurium, blood vessels and connective tissue, and cut into 1 mm³ explant pieces. The pieces are cultured in Dulbecco's Modified Easgle's Medium (DMEM) with 10% Fetal Bovine Serum (FBS), 100 U/ml penicillin, and 100 µg/ml streptomycin (base media) on tissue culture plastic dishes. Media is changed every 3 days, and explants are transferred to fresh dishes every 7 days for 4-6 weeks to allow for fibroblast migration out of the explants. Once fibroblast migration is complete, explants are digested with 0.3% trypsin-EDTA, 0.1% collagenase IV and 0.1% hyaluronidase at 37°C for 1 h, and dissociated by trituration. Dissociated Schwann cells are cultured on dishes coated with 100 µg/mL poly-L-lysine (PLL) in base media supplemented with 4 mM L-glutamine, 2 µM forskolin, 10 µg/mL bovine pituitary extract.

The Schwann cells were plated at a density of 30,000 cells/ml on the laminin micropatterned coverslips made as described in (i) above and cultured in SC media for 5-7 days to reach the desired level of cell density. Cell alignment to the stripes is measured in fixed cultures using a DAPI stain, where orientation of individual Schwann cells was determined from the orientation of their nuclei, which have ellipsoid shapes that align in the direction of their cellular extensions. In order to use the aligned Schwann cells as templates for replica molding, the samples must undergo a series of fixation steps. Samples were fixed with 2% paraformaldehyde, 4% sucrose, in 0.1M phosphate buffered saline pH 7.4 (PBS) for 15 min at room temperature, rinsed with PBS, then incubated in Karnovsky's fixative overnight. Samples were then rinsed with 0.1M cacodylate buffer, incubated in 1% OsO₄ in 0.1M cacodylate buffer for 1 h, rinsed with dH₂O, incubated in 1% thiocarbohydrazide in dH₂O for 1 h, incubated in 0.5% OsO₄ in d H₂O for 30 min, dehydrated with graded ethanols to 100% ethanol, and air-dried.

### (iv) SMC cell templating

Embryonic rat aortic smooth muscle cells (SMC) are obtained from the American Type Culture Collection (CRL-1444). SMC are cultured on flasks coated with 100µg/ml PLL in base medium supplemented with 4 mM L-glutamine at 37°C and 5% Co₂. SMC are rinsed with HBSS-CMF, treated with 0.25% trypsin-EDTA for 10 minutes to detach cells, resuspended in media, and plated on glass slides coated with 100 µg/ml PLL. Cultures are fixed following the same procedure as outlined for Schwann cells above.

### Example 2. Fabrication of patterned molds from cellular template

### (i) Fabrication of PDSM molds

PDMS is prepared using Sylgard® 184 from Dow Corning. Elastomer is combined with curing agent at a ratio of 10:1 elastomer base : curing agent and mixed. De-gassed PDMS is poured onto the fixed Schwann cells to form a layer 1-3 mm, degassed again for approximately 20 minutes, and cured on a hotplate at 95°C for 1 hour. After removing from the hotplate, PDMS is peeled off. This film substrate (PDMS1) contains indented regions that replicate the inverse of the cell morphology. To generate a film substrate that contains protruding regions that replicate the cell morphology (PDMS2), fresh PDMS is prepared, poured onto the PDMS1, and removed as described above. Of note, PDSM1 remains intact following its use as a template and it can be re-used either as a template for additional PDSM2 samples or for future experiments.

### (ii) Fabrication of PU molds

Polyurethane (PU) is prepared using PC-3585A carbothane solution (Thermedics Polymer Products). A 5% weight/volume of the solution in chloroform (Sigma) is heated to 65°C until clear, and entrapped air bubbles are removed by bath-sonication. The polymer mixture is applied to the cell template to form a layer of 1-1.5 mm. The solvent is allowed to evaporate and the resulting polymer film is removed. As is the case with the PDSM1 substrate, this substrate (PU1) contains indented regions that replicate the inverse of the cell morphology and may be employed in experiments or used as a template to generate a substrate that contains protruding regions (PU2).

### Example 3. In vitro stimulation of nerve growth

Cell cultures are incubated at 37°C and 5% CO₂ in a humidified environment. For culture on the cell templated substrates, cells are initially plated at specified densities onto the substrates in 100 µl of appropriate plating medium. After cells adhere to substrates for 3 h, 3 ml of appropriate base culture media are added as described in Example 1. DRG are grown in "neuronal media", which is base media supplemented with 50 ng/ml Nerve Growth Factor (NGF)..

The analysis of guided and stimulated cell growth employing the PDMS patterned molds from the cellular templates can be carried out using the same techniques, materials and methods described in detail in Goldner, et al, Biomaterials 27 (2006) 460-472.

### (i) Motion tracking analysis of DRG cells

Two aligned SC PDMS1 and two aligned SC PDSM2 cell templated films are coated with PLL/LN for 1 h using standard methods. A flat film of PDMS was used as a control and likewise coated. Thereafter, three films are seeded with 5000 DRG neurons in 100 µl drops and three films are seeded with 100,000 DRG neurons in 100 µl drops. The films are incubated at 37C, 5%CO2 for two hours and then 4 ml neuronal media (see Goldner, et al, Biomaterials 27 (2006) 460-72) is added to each film. Time lapse photography is then employed to track the motion of the cells on the films over a period of about 12 hours. A photographic image is taken of the films every 10 minutes during the period and graphically interpreted by taking each frame and separately recording the X and Y coordinates of a cell body using ImageJ, an image analysis tool available from the United States National Institutes of Health. The resulting list of coordinates enables calculation of the change in position of the cell bodies in terms of direction, distance, and speed from frame to frame. To generate the graph, the speed of each cell body on 4 adjacent fields of view (FOV) on one specific substrate type during the entire recording interval is averaged. The resulting number reflects how fast an average cell will move on a given substrate. In this Example, DRGs show a significantly higher average velocity on patterned substrates when compared to flat control substrates. (t test: 2-sample assuming unequal variances with alpha set at 0.05: adhesion difference between all groups significant). These results are shown in Figure 5.

### (ii) Alignment of cells on SC PDMS cell templated films

Two groups of SC PDMS1 and SC PDMS2 cell templated films are made as described in Example 2. The PDMS1 and PDMS2 films are seeded with 100,000 DRG neurons each. As control, flat PDMS films are seeded with 100,000 DRG neurons. The three groups of films are grown for 5 days in neuronal media and then analyzed for alignment. To determine alignment, the cells are stained with DAPI and phalloidin following standard methods, and 10 FOV are recorded for phase, DAPI, and actin. The DAPI results are imported into Image J, converted to 8-bit, thresh-holded to capture only the cell nuclei, and analyzed using ImageJ's particle analyzer. The software fits ellipses to the nuclei and determines the angle of the long axis of each ellipse. The results are exported into Excel and analyzed using circular distribution statistics software (Oriana, Kovach Computing Services). The results are shown in Figure 6. Figure 6A. shows a circular histogram of the long axes of the nuclei of DRG cells grown on PDMS1, PDMS2, and flat control substrates. The results visualize the strong and significantly different alignment of cellular growth on patterned substrates. Figure 6B, C, and D. are xerographic reproductions of photographic images taken of the films at day 5, shown the results represented in Fig 6A. Confirming those results it can be seen that the cells grown on the control films shown no alignment, whereas the cells grown on the both cell-templated films are aligned. In other words, the patterned substrates are very capable of aligning primary cell growth.

### (iii) Adhesion of DRG neurons on PDMS films

Nine samples are prepared as described in Example 3(i): 3 flat PDMS as control, 3 PDMS1 and 3 PDMS2, each featuring aligned SCs. With a circular hole punch 1.5 cm diameter circles are cut out of the samples and adhered to a 12 well sample dish with silicone grease. Next, 100 µL droplets of 100,000 cells/ml are suspended on the circles, totaling 10,000 DRG cells per circle in neuronal media. The circles are incubated for 1 h, then 2 ml neuronal media is added. Cells are fixed after 24 h and stained with DAPI. 10 FOV at 100x magnification are collected per circle, on patterned areas if applicable, and total muclei are counted with ImageJ.

### Example 4. In vivo use of cell templated conduit in nerve grafting in Lewis rats

50 Lewis rats (200-250g)(Harlan, WI) are divided evenly into 5 groups, with Group 1 animals receiving a 12 mm reversed isograft into the right sciatic nerve from Lewis donors, Group 2 animals receiving a 12 mm length PDMS conduit patterned by Schwann cells as described in Example 2, Group 3 animals receiving a 12 mm length unpatterned PDMS conduit; Group 4 animals receiving a 12 mm length PDMS conduit patterned by Schwann cells and seeded with laminin and Group 5 animals receiving a 12 mm length unpatterned PDMS conduit seeded with laminin. The animals are anesthetized and maintained by a 0.4 cc intramuscular injection of a premixed solution containing ketamine HCL, xylazine and atropine sulfate as described in Evans, Biomaterials 23 (2002) 841-48. Skin from the clipped lateral thigh is scrubbed with antiseptic solution and an incision extending distally from the greater trochanter to the midcalf is made to expose the sciatic and posterior tibial nerves. The sciatic nerve is divided near its origin and the 12 mm PDMS conduits or reversed isografts are sutured into the nerve gap using nylon sutures under microsurgical technique. Approximately 1 mm of the proximal and distal nerves are anchored into the conduit ends with sutures, leaving a 10 mm nerve gap following the methods disclosed in Evans, *supra.* Functional and histologic evaluations are conducted as in Evans, *supra.*

### Example 5. Preparation of cell templated bladder smooth muscle cells

Cell templated substrates are prepared as described in Examples 1 and 2. Briefly, ovine bladder smooth muscle cells are isolated from neonatal ovine bladder muscularis as described in Haberstroh et al., J. Urol. 162 (1999) 2114-18. The cells are maintained in DMEM (Dulbecco's Modified Eagle Medium) (Hyclone) supplemented with 10% fetal bovine serum (Hyclone) and 1% penicillin/streptomycin (Hycolone) under standard cell culture conditions as described in Thapa et al., Biomaterials 24 (2003) 2915-26.

The cells are seeded onto etched glass coverslips at a concentration of 3500 cell/cm² and are incubated under standard cell culture conditions in the culture media for 4 h. Cells are then rinsed in PBS, incubated in 2% paraformaldehyde with 4% sucrose for 15 minutes, rinsed in PBS, and incubated in Karnovsky's fixative for 3 hours - overnight. Cells are rinsed with 0.1M sodium cacodylate buffer, postfixed in 1% OsO₄ in cacodylate buffer for 1 hour, rinsed in dH₂O, and incubated in 1% thiocarbohydrazide in dH₂O for 30 minutes. Cells are dehydrated for 10 minutes each in 50%, 70%, 90%, and 100% ethanol, dried, and sputter coated with gold-palladium

PDMS is prepared using Sylgard® 184 from Dow Corning. Elastomer is combined with curing agent at a ratio of 10:1 elastomer base : curing agent and mixed. De-gassed PDMS is poured onto the fixed smooth muscle cells, de-gassed again for approximately 20 minutes, and cured on a hotplate at 95°C for 4-8 hours. After removing from the hotplate, the PDMS is peeled off. As described in Example 1, the smooth muscle templated PDSM1 substrate contains indented regions that replicate the inverse of the cell morphology. To generate a substrate that contains protruding regions that replicate the cell morphology (PDMS2), fresh PDMS is prepared, poured onto the smooth muscle templated PDMS1, and removed as described above.

Cell cultures are incubated at 37°C and 5% CO₂ in a humidified environment. For culture on the cell templated substrates (PDMS1 and PDMS2), cells are initially plated at specified densities onto the substrates in 100 µl of appropriate plating medium. Culture media composed of DMEM (Hyclone) with 10% fetal bovine serum (Hyclone) and penicillin/streptomycin (Hyclone) is then added.

The analysis of guided and stimulated cell growth employing the smooth muscles cell PDMS patterned molds from the cellular templates can be carried out using the same techniques, materials and methods described in detail in Goldner et al., Biomaterials 27 (2006) 460-472.

### DISCUSSION AND SUMMARY

In the above examples we describe the generation of biomimetic polymer materials with surfaces that contain replicas of cellular topography. Two distinct types of materials are produced: the impression replica containing indented topographical features and the relief replica containing protruding topographical features. Initially re-aligned Schwann cells were used as the source of cellular topography. However, there is no limit to the type of cell that can be employed and the procedures described apply to any of the cell types contemplated for use as templates.

The impression replica is prepared in a four-stage process, and the relief replica is prepared in a five-stage process, requiring one additional step beyond the preparation of the impression replica. As depicted in Figure 1, the stages include (1) photolithographic production of a polymeric stamp for micro-contact printing (µCP); (2) µCP of a micropatterned laminin-striped glass coverslip; (3) cell culture to generate a cell template containing fixed, aligned SC or other cells requiring alignment; (4) fabrication of the impression replica with indented topographical features; and (5) fabrication of the relief replica with protruding topographical features. The first three stages of the fabrication process are performed to align the cells; when using other cell types for applications where alignment is not desired, the cell template can be prepared simply by fixing the cultured cells. Use of a strong fixation procedure, similar to that required for SEM, preserves the cell morphology and allows the cells to act as a template. To generate the impression replica, which contains a replica of cellular topographical features indented into its surface, a solution containing the desired polymer is applied to the fixed cell sample. Once the solution has polymerized or phase separated, the impression replica is removed. A similar procedure is used to generate the relief replica, which contains a replica of cellular topographical features protruding from its surface. A solution containing the desired polymer is applied to the patterned surface of the impression replica, thus using the impression replica as a template. Once the solution has polymerized or phase separated, the relief replica is removed. Of note, the cell template, the impression replica, and the relief replica can all be re-used as templates for subsequent experiments.

Figures 2-4 summarize the results of the SC and SMC cell templating experiments using PDMS and PU as the polymers. Examination of corresponding regions of the SC cell template (Figure 2A-C), the impression replica (Figure 2D-F), and the relief replica (Figure 2G-I) under SEM demonstrates that multiple features can be replicated from the cell template, including: cell organization and alignment (Figure 2A, D, G), cell morphology (Figure 2A-I), and sub-cellular features (Figure 2C, F, I). At 1800x magnification (Figure 2C, F, I), the detailed morphological features of somata and cell extensions are visible and reproduced clearly in both replicas. Inherent to the replica process, the impression replica contains features that are mirrored with respect to both the cell template and the relief replica, since the impression replica is cast from the cell template and subsequently functions as the template for the relief replica.

Polymeric materials with surface replicas of nanoscale features can be achieved with this technique. Comparison of surface features between corresponding regions of the SC cell template (Figure 3A-C), the impression replica (Figure 3D-F), and the relief replica (Figure 3G-I) with WIM analysis confirms that feature geometry and dimensions are reproduced by the replica processing. The shapes, lengths, widths, and heights (or depths for the impression replica) of the cell somata are reproduced with this fabrication method, as measured and shown in the WIM oblique images (Figure 3A, D, G) and overhead images (Figure 3B, E, H). Shapes and x- and z-dimensions of cell extensions are replicated accurately to within 5-10 nm, as demonstrated by the corresponding surface profiles (Figure 3C, F, I) measured along the lines overlaid on the top views of Figures 3B, E, and H.

Various polymers can be employed using this approach to generate a wide range of material properties. For example, SC cell templates containing cellular processes of approximately 100nm in width were replicated in polyurethane (PU) (Figure 4A,B). Polymers that are useful for this technique are flowable in the liquid phase, capable of conversion to a rubbery or gelled solid upon curing or phase separation, detachable from a cell template, and capable of maintaining the cell-templated dimensions and geometry upon detachment from the cell template.

There is no limit to the type of cell whose topographical features can be replicated with this technique. In addition to SC, smooth muscle cells have served as cell templates (Figure 4C), with their features replicated in the impression replica (Figure 4D) and the relief replica (Figure 4E). In general, adherent cells can be utilized with the procedure described here, and cells that are cultured in suspension require just one additional step of immobilization on a solid material in order to function as a template.

Both PDMS and PU are biocompatible and support cell growth. The availability of two distinct types of surface replicas with indented cellular features - the impression replica, and protruding cellular features - the relief replica, opens up the possibility of a systematic investigation into the effects of cellular topography on cellular functions such as adhesion, orientation, activation, extension, and migration.

We have developed transparent, biomimetic surface replicas of mammalian cellular topography containing combinations of micron-scale and nanoscale features. With the incorporation of cellular topography onto surfaces, the roles of biomaterials can be expanded from providing simple mechanical support to providing critical cues for the study of cellular function. By combining materials that are biomimetic in cellular topography with technologies to modify bulk material properties, surface chemistry, and controlled release of diffusible factors, tailored biomaterial systems can be developed toward the ultimate goal of directing cells to form functional tissues.

Although the invention has been particularly described with reference to certain preferred embodiments, skilled artisans appreciate that changes in form and details may be made.

## Claims

1. A substrate for influencing the morphology, organization, spreading or adhesion of a cell to induce or stimulate growth, differentiation or regeneration of said cell or of a tissue that includes said cell, the substrate comprising a non-toxic, biocompatible film having a cell-templated morphology obtainable by the method of claim 3 formed from an elastomeric, polymeric gel or solid and having a surface patterned with features having dimensions and geometries that substantially reproduce the dimensions and geometries of a selected cell such that growth, differentiation or regeneration of said cell or tissue that includes said cell is induced or stimulated in the presence of said substrate, wherein the selected cell is a neuronal cell, a glial cell, a connective tissue cell, a muscle cell, an endothelial cell or a stem cell, provided the stem cell is not derived from a human embryo.

2. The substrate of claim 1, wherein:
a) the substrate is bioerodable and in the form of a tube or a channel having an interior surface from which the features of the substrate project either interiorly or exteriorly; and/or
b) the glial cell is a Schwann cell, an astrocyte, or an oligodendrocyte, or the connective tissue cell is a fibroblast cell, a myofibroblast cell or an osteoblast cell, or the muscle cell is a smooth muscle cell, a skeletal muscle cell, or a cardiac muscle cell; and/or
c) the elastomeric, polymeric gel or solid is formed from a curable polymer that is flowable in liquid phase, capable of conversion to a rubbery or gelled solid upon curing, detachable from a cell-template, and capable of maintaining the dimensions and geometries of the selected cell upon detachment from the cell-template; and in which case further optionally wherein:
i) the elastomeric polymeric gel or solid is an alkylsiloxane, a polylactic acid, a poly(D,L-lactide), a copolymer of lactic acid and glycolic acid, a copolymer of lactic acid and 0-aminocaproic acid, a polyhydroxylakanoate, a polyester, a polyglycolic acid, a polycaprolactone, a polydesoxazon, a copolymer of hydroxybutyric acid and hydroxyvaleric acid, a cross-linked hyaluronic acid, a poly(organo)phosphazine, a biodegradable polyurethane, a polyorthoester, a polyglycolic acid cross-linked to collagen, a copolymer of collagen and a glycosaminoglycan, a copolymer of L-lactide and ε-caprolactone, a mixture of polyurethane and polylactic acid, a mixture of a polymide and a polystyrene; a cross-liked hyaluronic acid, a poly(organo) phosphazane, a biodegradable polyurethane, and a fibrin glue, a polyethylene glycol (PEG) hydrogel, an agarose gel, a poly 2-hydroxyethylmethacrylate hydrogel, a poly N-(2-hydroxypropyl) methacrylamide hydrogel, a collagen gel, a chitosan gel, a gel mixture, a mixture of collagen, laminin and fibronectin, an alginate gel or a collagen-glycosaminoglycan gel; and/or
ii) the elastomeric polymeric gel or solid is polydimethylsiloxane, poly(ethoxymethylsiloxane) or a copolymer of lactic acid and glycolic acid.

3. A method of patterning the surface of an elastomeric polymer film substrate with dimensions and geometries that substantially reproduce the dimensions and geometries of a selected cell comprising the steps of forming a pre-polymer solution; placing in contact with the pre-polymer solution a cell-template composed of the selected cells; allowing the substrate to cure; and removing the substrate from the cell-template.

4. The method of claim 3:
a) further comprising the step of adding a bioactive molecule or guidance cue to the pre-polymer solution in order to assist in the growth and regeneration promotion; or
b) further comprising the step of coating the cell-templated substrate with a bioactive molecule or guidance cue after curing and removal of the substrate from the cell-template; or
c) wherein the cell-templated film comprises a bioerodable implant; and in which case optionally wherein the implant is formed in the shape of a tube or a channel with an interior surface from which the morphology of the template cell projects interiorly or exteriorly to create a patterned interior tube or channel surface that substantially reproduces the native growth and differentiation pattern of the selected cell.
d) wherein the selected cell is a neuronal cell, a glial cell, a connective tissue cell, a muscle cell an endothelial cell, or a stem cell provided the stem cell is not provided from a human embryo, and in which case optionally wherein the glial cell is a Schwann cell, an astrocyte, or an oligodendrocyte, or the connective tissue cell is a fibroblast cell, a myofibroblast cell or an osteoblast cell; or the muscle cell is a smooth muscle cell, a skeletal muscle cell, or a cardiac muscle cell, or
e) wherein the cell-templated film is composed of an elastomeric, natural or synthetic, polymeric gel or solid; and in which case optionally wherein the elastomeric polymeric gel or solid cell-templated film is formed from a curable polymer that is flowable in liquid phase, capable of conversion to a rubbery or gelled sold upon curing, detachable from a cell-template, and capable of maintaining the cell-templated dimensions and geometry upon detachment from the cell-template; and in which case further optionally wherein the elastomeric polymer film substrate is an alkysiloxane, a polylactic acid, a poly(D,L-lactide), a copolymer of lactic acid and glycolic acid, a copolymer of lactic acid and 0-aminocaproic acid, a polyhydroxylakanoate, a polyester, a polyglycolic acid, a polycaprolactone, a polydesoxazon, a copolymer of hydroxybutyric acid and hydroxyvaleric acid, a cross-linked hyaluronic acid, a poly(organo)phosphazine, a biodegradable polyurethane, a polyorthoester, a polyglycolic acid cross-linked to collagen, a copolymer of collagen and a glycosaminoglycan, a copolymer of L-lactide and ε-caprolactone, a mixture of polyurethane and polylactic acid, a mixture of a polyimide and a polystyrene; a cross-linked hyaluronic acid, a poly(organo) phosphazane, a biodegradable polyurethane, and a fibrin glue, a polyethylene glycol (PEG) hydrogel, an agarose gel, a poly 2-hydroxyethylmethacrylate hydrogel, a poly N-(2-hydroxypropyl) methacrylamide hydrogel, a collagen gel, a chitosan gel, and a gel mixture such as a mixture of collagen, laminin and fibronectin, an alginate gel and a collagen-glycosaminoglycan gel; and in which case further optionally wherein the gel or sold film is polydimethylsiloxane, poly(ethoxymethylsiloxane) or a copolymer of lactic acid and glycolic acid.

5. A cell-templated substrate for use in repairing injured cells or tissues of a selected cell type in a mammalian patient, the cell-templated substrate comprising a non-toxic, biocompatible film having a cell-templated morphology obtainable by the method of claim 3 formed of an elastomeric, polymeric gel or solid and having a surface patterned with features having dimensions and geometries that substantially reproduce the dimensions and geometries of a neuronal cell, a glial cell, a connective tissue cell, a muscle cell, an endothelial cell or a stem cell, wherein the cell-templated substrate is, in use, positioned in proximity to the injured cells or tissues to allow new cells or tissues of the selected cell type to grow onto the cell-templated substrate, provided the stem cell is not provided from a human embryo.

6. The cell-templated substrate for use in repairing injured cells or tissues of a selacted cell type in a mammalian patient of claim 5, wherein:
a) one or more bioactive molecules or guidance cues is coated on the cell-templated substrate prior to positioning the substrate in proximity to the injured cells or tissues; or
b) the cell-templated substrate is bioerodable and is formed in the shape of a tube or channel having an interior surface from which the features of the cell-templated substrate project either exteriorly or interiorly; and in which case optionally wherein the elastomeric polymeric gel or solid is a polydimethylsiloxane or a poly(ethoxymethlsiloxane).

7. A cell-templated conduit for use in regenerating a severed or damaged nerve, wherein the cell-templated conduit is formed from a non-toxic, biocompatible film having a cell-templated morphology obtainable by the method of claim 3 formed from an elastomeric, polymeric gel or solid and has an interior surface patterned with features having dimensions and geometries that substantially reproduce dimensions and geometries of a neuronal cell or a glial cell and projecting either exteriorly or interiorly from the interior surface of the cell-templated conduit, and wherein the cell-templated conduit, in use, is positioned in proximity to an end of the severed or a selected cell type in a mammalian patient damaged nerve such that the severed or damaged nerve grows into the cell-templated conduit.

8. The cell-templated conduit for use in regenerating a several or damaged nerve of claim 7, wherein the interior surface of the cell-templated conduit is coated with a nerve growth guidance cue, and in which case optionally wherein the nerve growth guidance cue is laminin or nerve growth factor (NGF).

## Patentansprüche

1. Substrat zum Beeinflussen der Morphologie, Organisation, Verteilung oder Adhäsion einer Zelle, um ein Wachstum, eine Differenzierung oder eine Regeneration der Zelle oder eines die Zelle enthaltenden Gewebes zu induzieren oder stimulieren, wobei das Substrat einen nicht-toxischen, biologisch kompatiblen Film mit einer zellschablonierten Morphologie umfasst, der mithilfe des Verfahrens nach Anspruch 3 erhältlich und aus einem elastomeren, polymeren Gel oder Feststoff gebildet ist und eine mit Merkmalen strukturierte Oberfläche aufweist, die Dimensionen und Geometrien haben, die die Dimensionen und Geometrien einer ausgewählten Zelle im Wesentlichen reproduzieren, so dass ein Wachstum, eine Differenzierung oder eine Regeneration der Zelle oder des Gewebes in der Gegenwart des Substrats induziert oder stimuliert wird, wobei die ausgewählte Zelle eine Nervenzelle, eine Gliazelle, eine Bindegewebezelle, eine Muskelzelle, eine Endothelzelle oder eine Stammzelle ist, mit der Maßgabe, dass die Stammzelle nicht von einem menschlichen Embryo abgeleitet ist.

2. Substrat nach Anspruch 1, wobei:
a) das Substrat biologisch erodierbar ist und in Form eines Rohrs oder Kanals mit einer Innenfläche vorliegt, von der die Merkmale des Substrats nach innen oder nach außen vorspringen; und/oder
b) die Glialzelle eine Schwann-Zelle, ein Astrozyt oder ein Oligodendrozyt ist, oder die Bindegewebezelle eine Fibroblastenzelle, eine Myofibroblastenzelle oder eine Osteoblastenzelle ist, oder die Muskelzelle eine glatte Muskelzelle, eine Skelettmuskelzelle oder eine Herzmuskelzelle ist; und/oder
c) das elastomere, polymere Gel oder der elastomere, polymere Feststoff aus einem härtbaren Polymer gebildet ist, das in Flüssigphase fließbar ist, das sich bei Härtung in einen gummiartigen oder gelierten Feststoff verwandeln kann, das von einer Zellschablone lösbar ist und das die Dimensionen und Geometrien der ausgewählten Zelle bei Loslösung von der Zellschablone beibehalten kann; und wobei in diesem Fall darüber hinaus optional:
i) das elastomere, polymere Gel oder der elastomere, polymere Feststoff ein Alkylsiloxan, eine Polymilchsäure, ein Poly(D,L-Lactid), ein Copolymer von Milchsäure und Glykolsäure, ein Copolymer von Milchsäure und ε-Aminocapronsäure, ein Polyhydroxylakanoat, ein Polyester, eine Polyglykolsäure, ein Polycaprolacton, ein Polydesoxazon, ein Copolymer von Hydroxybuttersäure und Hydroxyvaleriansäure, eine vernetzte Hyaluronsäure, ein Poly(organo)phosphazin, ein biologisch abbaubares Polyurethan, ein Polyorthoester, eine mit Kollagen vernetzte Polyglykolsäure, ein Copolymer von Kollagen und Glycosaminoglykan, ein Copolymer von L-Lactid und ε-Caprolacton, eine Mischung von Polyurethan und Polymilchsäure, eine Mischung eines Polymids und eines Polystyrols; eine vernetzte Hyaluronsäure, ein Poly(organo)phosphazan, ein biologisch abbaubares Polyurethan und ein Fibrinklebstoff, ein Polyethylenglykol-(PEG)-Hydrogel, ein Agarosegel, ein Poly-2-hydroxyethylmethacrylat-Hydrogel, ein Poly-N-(2-hydroxypropyl)methacrylamid-Hydrogel, ein Kollagengel, ein Chitosangel, eine Gelmischung, eine Mischung von Kollagen, Laminin und Fibronektin, ein Alginatgel oder ein Kollagen-Glycosaminoglykan-Gel ist; und/oder
ii) das elastomere, polymere Gel oder der elastomere, polymere Feststoff Polydimethylsiloxan, Poly(ethoxymethylsiloxan) oder ein Copolymer von Milchsäure und Glykolsäure ist.

3. Verfahren zum Strukturieren der Oberfläche eines elastomeren Polymerfilmsubstrats mit Dimensionen und Geometrien, die im Wesentlichen die Dimensionen und Geometrien einer ausgewählten Zelle reproduzieren, das die Schritte des Bildens einer Vorpolymerlösung; des Bringens einer Zellschablone, die aus den ausgewählten Zellen zusammengesetzt ist, in Kontakt mit der Vorpolymerlösung; des Zulassens, dass das Substrat härtet; und des Entfernens des Substrat von der Zellschablone.

4. Verfahren nach Anspruch 3:
a) das darüber hinaus den Schritt des Hinzufügens eines biologisch aktiven Moleküls oder Wegweisermoleküls zur Vorpolymerlösung umfasst, um die Wachstums- und Regenerationsförderung zu unterstützen; oder
b) das darüber hinaus den Schritt des Beschichtens des zellschablonierten Substrats mit einem biologisch aktiven Molekül oder Wegweisermolekül nach dem Härten und Entfernen des Substrats von der Zellschablone umfasst; oder
c) wobei der zellschablonierte Film ein biologisch erodierbares Implantat umfasst; und wobei in diesem Fall das Implantat optional in die Form eines Rohrs oder eines Kanals mit einer Innenfläche geformt ist, von der die Morphologie der Schablonenzelle nach innen oder nach außen vorspringt, um eine strukturierte Innenrohr- oder Innenkanalfläche zu bilden, die das native Wachstums- und Differenzierungsmuster der ausgewählten Zelle im Wesentlichen reproduziert,
d) wobei die ausgewählte Zelle eine Nervenzelle, eine Glialzelle, eine Bindegewebezelle, eine Muskelzelle, eine Endothelzelle oder eine Stammzelle ist, mit der Maßgabe, dass die Stammzelle nicht von einem menschlichen Embryo abgeleitet ist, und optional wobei in diesem Fall die Glialzelle ein Schwann-Zelle, ein Astrozyt oder ein Oligodendrozyt ist oder die Bindegewebezelle eine Fibroblastenzelle, eine Myofibroblastenzelle oder eine Osteoblastenzelle ist; oder die Muskelzelle eine glatte Muskelzelle, eine Skelettmuskelzelle oder eine Herzmuskelzelle ist, oder
e) wobei der zellschablonierte Film aus einem elastomeren, natürlichen oder synthetischen, polymeren Gel oder Feststoff zusammengesetzt ist; und optional wobei in diesem Fall der zellschablonierte Film aus elastomerem, polymerem Gel oder Feststoff aus einem härtbaren Polymer gebildet ist, das in Flüssigphase fließbar ist, das sich bei Härtung in einen gummiartigen oder gelierten Feststoff verwandeln kann, das von einer Zellschablone lösbar ist und das die Dimensionen und Geometrien der ausgewählten Zelle bei Loslösung von der Zellschablone beibehalten kann; und wobei in diesem Fall darüber hinaus optional das elastomere, polymere Filmsubstrat ein Alkylsiloxan, eine Polymilchsäure, ein Poly(D,L-Lactid), ein Copolymer von Milchsäure und Glykolsäure, ein Copolymer von Milchsäure und ε-Aminocapronsäure, ein Polyhydroxylakanoat, ein Polyester, eine Polyglykolsäure, ein Polycaprolacton, ein Polydesoxazon, ein Copolymer von Hydroxybuttersäure und Hydroxyvaleriansäure, eine vernetzte Hyaluronsäure, ein Poly(organo)phosphazin, ein biologisch abbaubares Polyurethan, ein Polyorthoester, eine mit Kollagen vernetzte Polyglykolsäure, ein Copolymer von Kollagen und Glycosaminoglykan, ein Copolymer von L-Lactid und ε-Caprolacton, eine Mischung von Polyurethan und Polymilchsäure, eine Mischung eines Polyimids und eines Polystyrols; eine vernetzte Hyaluronsäure, ein Poly(organo)phosphazan, ein biologisch abbaubares Polyurethan und ein Fibrinklebstoff, ein Polyethylenglykol-(PEG)-Hydrogel, ein Agarosegel, ein Poly-2-Hydroxyethylmethacrylat-Hydrogel, ein Poly-N-(2-hydroxypropyl)methacrylamid-Hydrogel, ein Kollagengel, ein Chitosangel und eine Gelmischung, z. B. eine Mischung von Kollagen, Laminin und Fibronektin, ein Alginatgel und ein Kollagen-Glycosaminoglykan-Gel ist; und darüber hinaus optional wobei in diesem Fall der Gel- oder Feststofffilm Polydimethylsiloxan, Poly(ethoxymethylsiloxane) oder ein Copolymer von Milchsäure und Glykolsäure ist.

5. Zellschabloniertes Substrat zur Verwendung bei der Wiederherstellung von verletzten Zellen oder Geweben eines ausgewählten Zelltyps bei einem Säugetier-Patienten, wobei das zellschablonierte Substrat einen nicht-toxischen, biologisch kompatiblen Film mit einer zellschablonierten Morphologie umfasst, der mithilfe des Verfahrens nach Anspruch 3 erhältlich und aus einem elastomeren, polymeren Gel oder Feststoff gebildet ist und eine mit Merkmalen strukturierte Oberfläche aufweist, die Dimensionen und Geometrien haben, die die Dimensionen und Geometrien einer Nervenzelle, einer Glialzelle, einer Bindegewebezelle, einer Muskelzelle, einer Endothelzelle oder einer Stammzelle im Wesentlichen reproduzieren, wobei das zellschablonierte Substrat in Verwendung in der Nähe der verletzten Zellen oder Gewebe positioniert ist, um zu ermöglichen, dass neue Zellen oder Gewebe des ausgewählten Zelltyps auf das zellschablonierte Substrat wachsen, mit der Maßgabe, dass die Stammzelle nicht von einem menschlichen Embryo abgeleitet ist.

6. Zellschabloniertes Substrat zur Verwendung bei der Wiederherstellung von verletzten Zellen oder verletztem Gewebe eines ausgewählten Zelltyps bei einem Säugetier-Patienten nach Anspruch 5, wobei
a) ein oder mehrere biologisch aktive Moleküle oder Wegweisermoleküle vor dem Positionieren des Substrats in der Nähe der verletzten Zellen oder Geweben auf das zellschablonierte Substrat beschichtet wird; oder
b) das zellschablonierte Substrat biologisch erodierbar und in der Form eines Rohrs oder eines Kanals mit einer Innenfläche geformt ist, von der die Merkmale des zellschablonierten Substrats nach außen oder nach innen vorspringen; und wobei in diesem Fall das elastomere, polymere Gel oder der elastomere, polymere Feststoff optional Polydimethylsiloxan oder ein Poly(ethoxymethylsiloxan) ist.

7. Zellschablonierte Leitkanal zur Verwendung bei der Regeneration eines durchtrennten oder beschädigten Nervs, wobei der zellschablonierte Leitkanals aus einem nicht-toxischen, biologisch kompatiblen Film mit einer zellschablonierten Morphologie gebildet ist, der mithilfe des Verfahrens nach Anspruch 3 erhältlich und aus einem elastomeren, polymeren Gel oder Feststoff gebildet ist und eine mit Merkmalen strukturierte Innenfläche aufweist, die Dimensionen und Geometrien haben, die Dimensionen und Geometrien einer Nervenzelle oder einer Glialzelle reproduzieren und die von der Innenfläche des zellschablonierten Leitkanals nach außen oder nach innen vorspringen, und wobei der zellschablonierte Leitkanal in Gebrauch in der Nähe eines Endes des durchtrennten oder beschädigten Nervs positioniert ist, so dass der durchtrennte oder geschädigte Nerv in den zellschablonierten Leitkanal wächst.

8. Zellschablonierter Leitkanal zur Verwendung bei der Regeneration eines durchtrennten oder beschädigten Nervs nach Anspruch 7, wobei die Innenfläche des zellschablonierten Leitkanals mit einem Nervenwachstums-Wegweisermolekül beschichtet ist, und darüber hinaus wobei das Nervenwachstums-Wegweisermolekül in diesem Fall Laminin oder Nervenwachstumsfaktor (NGF) ist.

## Revendications

1. Substrat pour influencer la morphologie, l'organisation, la propagation ou l'adhérence d'une cellule pour induire ou stimuler la croissance, la différenciation ou la régénération de ladite cellule ou d'un tissu qui comprend ladite cellule, le substrat comprenant un film biocompatible, non toxique, ayant une morphologie à matrice cellulaire pouvant être obtenue par le procédé de la revendication 3, formé à partir d'un gel ou solide polymère, élastomère, et ayant une surface à motif avec des caractéristiques ayant des dimensions et des géométries qui reproduisent de façon substantielle les dimensions et les géométries d'une cellule choisie de telle sorte que la croissance, la différenciation ou la régénération de ladite cellule ou dudit tissu qui comprend ladite cellule est induite ou stimulée en présence dudit substrat, la cellule choisie étant une cellule neuronale, une cellule gliale, une cellule de tissu conjonctif, une cellule de muscle, une cellule endothéliale ou une cellule souche, à la condition que la cellule souche ne soit pas issue d'un embryon humain.

2. Substrat selon la revendication 1, dans lequel :
a) le substrat est bioérodable et dans la forme d'un tube ou d'un canal ayant une surface intérieure à partir de laquelle les caractéristiques du substrat se projettent soit intérieurement soit extérieurement ; et/ou
b) la cellule gliale est une cellule de Schwann, un astrocyte ou un oligodendrocyte, ou la cellule de tissu conjonctif est une cellule fibroblaste, une cellule myofibroblaste ou une cellule ostéoblaste, ou la cellule de muscle est une cellule de muscle lisse, une cellule de muscle squelettique ou une cellule de muscle cardiaque ; et/ou
c) le gel ou solide polymère, élastomère, est formé à partir d'un polymère durcissable qui est capable de s'écouler en phase liquide, apte à se convertir en un solide caoutchouteux ou gélifié lors du durcissement, détachable à partir d'une matrice cellulaire et apte à maintenir les dimensions et les géométries de la cellule choisie lors du détachement de la matrice cellulaire ; auquel cas encore facultativement dans lequel :
i) le gel ou solide polymère, élastomère, est un alkylsiloxane, un acide polylactique, un poly(D,L-lactide), un copolymère d'acide lactique et d'acide glycolique, un copolymère d'acide lactique et d'acide ε-aminocaproïque, un polyhydroxylalcanoate, un polyester, un acide polyglycolique, une polycaprolactone, un polydésoxazon, un copolymère d'acide hydroxybutyrique et d'acide hydroxyvalérique, un acide hyaluronique réticulé, une poly(organo)phosphazine, un polyuréthane biodégradable, un polyorthoester, un acide polyglycolique réticulé à du collagène, un copolymère de collagène et d'un glycosaminoglycane, un copolymère de L-lactide et d'ε-caprolactone, un mélange de polyuréthane et d'acide polylactique, un mélange d'un polyimide et d'un polystyrène ; un acide hyaluronique réticulé, un poly(organo)phosphazane, un polyuréthane biodégradable et une colle de fibrine, un hydrogel de polyéthylène glycol (PEG), un gel d'agarose, un hydrogel de poly(méthacrylate de 2-hydroxyéthyle), un hydrogel de poly(N-(2-hydroxypropyl)méthacrylamide), un gel de collagène, un gel de chitosane, un mélange de gel, un mélange de collagène, de laminine et de fibronectine, un gel d'alginate ou un gel de collagène-glycosaminoglycane ; et/ou
ii) le gel ou solide polymère, élastomère, est un polydiméthylsiloxane, un poly(éthoxyméthylsiloxane) ou un copolymère d'acide lactique et d'acide glycolique.

3. Procédé de de formation de motif sur la surface d'un substrat de film polymère élastomère ayant des dimensions et des géométries qui reproduisent de façon substantielle les dimensions et les géométries d'une cellule choisie comprenant les étapes consistant à former une solution de prépolymère ; placer en contact avec la solution de prépolymère une matrice cellulaire composée des cellules choisies, amener le substrat à durcir ; et retirer le substrat de la matrice cellulaire.

4. Procédé selon la revendication 3 :
a) comprenant en outre l'étape d'addition d'une molécule bioactive ou d'un signal de guidage à la solution de prépolymère de façon à aider à favoriser la croissance et la régénération ; ou
b) comprenant en outre l'étape de revêtement du substrat à matrice cellulaire par une molécule bioactive ou un signal de guidage après durcissement et retrait du substrat à partir de la matrice cellulaire ; ou
c) dans lequel le film à matrice cellulaire comprend un implant bioérodable ; et auquel cas facultativement dans lequel l'implant est formé dans la forme d'un tube ou d'un canal ayant une surface intérieure à partir de laquelle la morphologie de la cellule de matrice se projette intérieurement ou extérieurement pour créer une surface de tube ou canal intérieur à motif qui reproduit de façon substantielle le motif de croissance et de différenciation endogène de la cellule choisie ;
d) dans lequel la cellule choisie est une cellule neuronale, une cellule gliale, une cellule de tissu conjonctif, une cellule de muscle, une cellule endothéliale ou une cellule souche, à la condition que la cellule souche ne soit pas issue d'un embryon humain, et auquel cas facultativement dans lequel la cellule gliale est une cellule de Schwann, un astrocyte ou un oligodendrocyte, ou la cellule de tissu conjonctif est une cellule fibroblaste, une cellule myofibroblaste ou une cellule ostéoblaste ; ou la cellule de muscle est une cellule de muscle lisse, une cellule de muscle squelettique ou une cellule de muscle cardiaque, ou
e) dans lequel le film à matrice cellulaire est composé d'un gel ou solide polymère, naturel ou synthétique, élastomère ; et auquel cas facultativement dans lequel le film à matrice cellulaire de gel ou solide polymère élastomère est formé à partir d'un polymère durcissable qui est capable de s'écouler en phase liquide, apte à se convertir en un solide caoutchouteux ou gélifié lors du durcissement, détachable d'une matrice cellulaire et apte à maintenir les dimensions et la géométrie de matrice cellulaire lors du détachement de la matrice cellulaire ; et auquel cas encore facultativement dans lequel le substrat de film polymère élastomère est un alkylsiloxane, un acide polylactique, un poly(D,L-lactide), un copolymère d'acide lactique et d'acide glycolique, un copolymère d'acide lactique et d'acide ε-aminocaproïque, un polyhydroxylalcanoate, un polyester, un acide polyglycolique, une polycaprolactone, un polydésoxazon, un copolymère d'acide hydroxybutyrique et d'acide hydroxyvalérique, un acide hyaluronique réticulé, une poly(organo)phosphazine, un polyuréthane biodégradable, un polyorthoester, un acide polyglycolique réticulé à du collagène, un copolymère de collagène et d'un glycosaminoglycane, un copolymère de L-lactide et d'ε-caprolactone, un mélange de polyuréthane et d'acide polylactique, un mélange d'un polyimide et d'un polystyrène ; un acide hyaluronique réticulé, un poly(organo)phosphazane, un polyuréthane biodégradable et une colle de fibrine, un hydrogel de polyéthylène glycol (PEG), un gel d'agarose, un hydrogel de poly(méthacrylate de 2-hydroxyéthyle), un hydrogel de poly(N-(2-hydroxypropyl)méthacrylamide), un gel de collagène, un gel de chitosane, et un mélange de gel tel qu'un mélange de collagène, de laminine et de fibronectine, un gel d'alginate ou un gel de collagène-glycosaminoglycane ; et auquel cas encore facultativement dans lequel le film de gel ou solide est un polydiméthylsiloxane, un poly(éthoxyméthylsiloxane) ou un copolymère d'acide lactique et d'acide glycolique.

5. Substrat à matrice cellulaire en vue d'une utilisation dans la réparation de cellules ou de tissus lésés d'un type cellulaire choisi chez un patient mammifère, le substrat à matrice cellulaire comprenant un film biocompatible, non toxique, ayant une morphologie à matrice cellulaire pouvant être obtenue par le procédé selon la revendication 3, formé à partir d'un gel ou solide polymère, élastomère, et ayant une surface à motif avec des caractéristiques ayant des dimensions et des géométries qui reproduisent de façon substantielle les dimensions et les géométries d'une cellule neuronale, d'une cellule gliale, d'une cellule de tissu conjonctif, d'une cellule de muscle, d'une cellule endothéliale ou d'une cellule souche, dans lequel le substrat à matrice cellulaire est, lors de l'utilisation, positionné à proximité des cellules ou tissus lésés pour permettre à de nouvelles cellules ou à de nouveaux tissus du type cellulaire choisi de croître sur le substrat à matrice cellulaire, à la condition que la cellule souche ne soit pas issue d'un embryon humain.

6. Substrat à matrice cellulaire en vue d'une utilisation dans la réparation de cellules ou de tissus lésés d'un type cellulaire choisi chez un patient mammifère, selon la revendication 5, dans lequel :
a) une ou plusieurs molécules bioactives ou signaux de guidage sont appliqués en revêtement sur le substrat à matrice cellulaire avant le positionnement du substrat à proximité des cellules ou tissus lésés ; ou
b) le substrat à matrice cellulaire est bioérodable et est formé dans la forme d'un tube ou canal ayant une surface intérieure à partir de laquelle les caractéristiques du substrat à matrice cellulaire se projettent soit extérieurement soit intérieurement ; et auquel cas facultativement dans lequel le gel ou solide polymère élastomère est un polydiméthylsiloxane ou un poly(éthoxyméthylsiloxane).

7. Conduit à matrice cellulaire en vue d'une utilisation dans la régénération d'un nerf coupé ou endommagé, dans lequel le conduit à matrice cellulaire est formé à partir d'un film biocompatible, non toxique, ayant une morphologie à matrice cellulaire pouvant être obtenue par le procédé selon la revendication 3, formé à partir d'un gel ou solide polymère, élastomère, et a une surface intérieure à motif avec des caractéristiques ayant des dimensions et des géométries qui reproduisent de façon substantielle les dimensions et les géométries d'une cellule neuronale ou d'une cellule gliale et se projetant soit extérieurement soit intérieurement à partir de la surface intérieure du conduit à matrice cellulaire, et dans lequel le conduit à matrice cellulaire, lors de l'utilisation, est positionné à proximité d'une extrémité du nerf coupé ou endommagé de telle sorte que le nerf coupé ou endommagé croisse dans le conduit à matrice cellulaire.

8. Conduit à matrice cellulaire en vue d'une utilisation dans la régénération d'un nerf coupé ou endommagé selon la revendication 7, dans lequel la surface intérieure du conduit à matrice cellulaire est revêtue par un signal de guidage de croissance de nerf, et auquel cas facultativement dans lequel le signal de guidage de croissance de nerf est la laminine ou le facteur de croissance du nerf (NGF).
